# EUROPEAN PATENT APPLICATION

(11) **EP 4 361 131 A1**
(43) Date of publication of application: **01.05.2024**
(21) Application number: 21946296.7
(22) Date of filing: 21.06.2021
(51) Int. Cl.: C07D 213/73, C07D 209/02, C12N 5/0735, A61K 31/4184, A61K 31/4375, A61P 35/00, A61P 9/00, A61P 9/10, A61P 3/10, A61P 3/04, A61P 25/28, A61P 25/00, A61P 21/00, A61P 37/06, A61P 19/02, A61P 17/14, A61P 17/02, A61P 27/00

(54) **OCT4 HIGH-SELECTIVITY ACTIVATOR**

(71) Applicant: Iregene Therapeutics Ltd, Hubei 430075 (CN)
(72) Inventor: WEI, Jun, Wuhan, Hubei 430075 (CN); CAI, Meng, Wuhan, Hubei 430075 (CN); YUN, Xuan, Wuhan, Hubei 430075 (CN)
(74) Representative: Petraz, Gilberto Luigi
(86) International application number: PCT/CN2021/101226
(87) International publication number: WO 2022/266794

(57) **Abstract**

A high-selectivity activator capable of being used for OCT4 and downstream gene expression, a pharmaceutical composition thereof, and a preparation method therefor. The activator has the following formula: 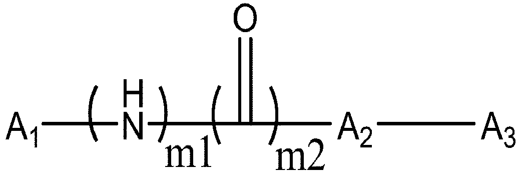 wherein, A₁, m1, m2, A₂ and A₃ are described in this paper.

## Description

### Background of the Invention

### Field of the Invention

The invention relates to the field of medicine, particularly an OCT4 high-selectivity activator, its pharmaceutical composition and preparation method, and its application in regulating OCT4 and its associated genes.

### Description of Related Art

Regenerative medicine refers to the emerging science of using various new technical disciplines to rebuild tissues and organs with aging or functional loss and to treat related diseases through various medical means. The important research direction of regenerative medicine is the mechanism of normal tissue characteristics and functions, the biological basis of post-traumatic repair, the regeneration mechanism of tissues and organs, and the differentiation mechanism of various stem cells to finally obtain effective biological treatment methods. Among them, Embryonic stem cells (ESCs, referred to as ES, EK, or ESC cells) are the most eye-catching cell types in early regenerative medicine research. However, the obtainment and use of the cells have great ethical controversy because researching embryonic stem cells requires the destruction of the embryo, which is the life form in the womb when a person is not yet formed. This ethical controversy has greatly hindered the advancement and application of regenerative medicine.

In 2006, Shinya Yamanaka's team invented a "cocktail" method composed of four transcription factors: OCT4, SOX2, KLF4, and c-Myc, which can successfully reprogram terminally differentiated skin fibroblasts into stem cells with differentiation pluripotency, which are called induced pluripotent stem cells (Takahashi K, et al., Cell, 2006, 126(4) pp.663-676; Takahashi K and Yamanaka S, Cell, 2007, 131(5) pp. 861-872). These stem cells have similar differentiation potential to embryonic stem cells. They can form the three most basic germ layers of human body development: ectoderm, mesoderm, and endoderm, and eventually form various adult cells. This invention breaks through the ethical restrictions on using human embryonic stem cells in medicine and dramatically expands the application potential of stem cell technology in clinical medicine.

In the study of induced Pluripotent Stem Cells and embryonic stem cells, OCT4 has been proven to be a major regulatory gene for reprogramming and induction of cell plasticity (Malik, V et al., Nat. Commun. 2019, 10, 3477). The protein encoded by the OCT4 gene plays a key role in embryonic development and stem cell pluripotency, and alternative splicing results in multiple transcript variants. The protein encoded by OCT4 belongs to the POU domain family of transcription factors, located in Chromosome 17:35,825,200-35,829,401. The hallmark feature of the POU transcription factor family is the POU domain, which consists of two structurally independent subdomains: a POU-specific (POU) region consisting of highly conserved 75-amino acid, and a carboxyl-terminal homology domain (POUh) consisting of 60-amino acid. The expression of OCT4 is under regulation at the transcriptional level by upstream cis-acting elements of the OCT4 gene and chromatin structure methylation (Klemm JD, et al., Cell, 1994, 77:21-32; Brehm A, et al., Mol Cell Biol 1997; 17:154-62). By analyzing the expression of the LacZ reporter gene under the control of an 18Kb fragment from the OCT4 genomic locus, Yeom et al. identified two elements. They named them the proximal enhancer (PE) and the distal enhancer (DE) that may need to be regulated. They identified the precise binding sites of transcription factors in these two enhancers. POU domain transcription factors bind to specific octameric DNA and regulate differentiation pathways of cell type specificity. Among them, during the formation of iPSCs, OCT4 containing the POU domain and Sox2 containing the HMG domain are transcription factors crucial for maintaining the pluripotency of pluripotent cells (Nichols, J., et al., Cell, 1998, 95, 379-391; Avilion, A., et al., 2003, Genes Dev.17, 126-140), at least part of their function in pluripotent cells is to drive the transcription of target genes through a cooperative interaction between the two (Tomioka, M., et al. Nucleic Acids Res. 2002; 30, 3202-3213). These findings suggest that developmental switching can be controlled by OCT4. At present, the reprogramming method widely used mostly uses viruses or other types of vectors to overexpress OCT4 (Takahashi K, et al., Cell, 2006, 126(4): 663-676; Takahashi K and Yamanaka S, Cell, 2007, 131(5):861-872); Yu J, et al. Science. 2007; 318:1917-1920). This method has potential clinical risks in the clinical use of induced Pluripotent Stem Cells (iPSC), such as the hidden danger of tumorigenicity brought about by the use of viral vectors. In addition, the complex GMP manufacturing process of the vector also brings complexity to the clinical regulation of induced Pluripotent Stem Cells. Further, such products will bring high costs due to the use of vectors.

Based on the above reasons, the present invention designs and uses benzimidazole derivatives and aminopyridine derivatives to achieve the expression regulation of their downstream genes through the chemical regulation of the OCT4 promoter. Thus, the regulation of OCT4 by viruses or other vectors is avoided, and the safe and simple chemical small molecules initiating biological expression function can be further achieved.

### Summary of the Invention

The present invention relates to the compound of the structure of formula (I) or its pharmaceutically acceptable salt, solvate, active metabolite, polymorph, ester, optical isomer or prodrug, pharmaceutical composition containing the compound of the structure of formula (I) and its use as an OCT4 high-selectivity activator for cell reprogramming.

The invention provides a compound of formula (I): 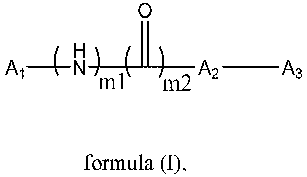 wherein:
A₁ is 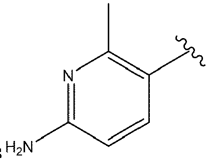 or 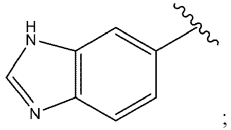
m1 and m2 are 0 or 1, respectively;
A₂ is C1-C6 alkylene, C2-C6 alkenylene, -O(CH₂)q-, -NR₁-, -SO₂-, -(CH₂)_{V}NHS(O)₂- or a bond, wherein q is 1 or 2 or 3 or 4, V is 0 or 1 or 2, and R₁ is selected from H or C1-C4 alkyl;
A₃ is C1-C6 alkyl; C2-C6 alkenyl; C4-C6 cycloalkyl, wherein one carbon atom can be replaced by N, O, S heteroatom; 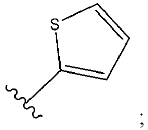 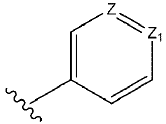 Z and Z₁ are N or CR₂, respectively, R₂ is selected from H, halogen, C1-C4 alkyl or cyano; 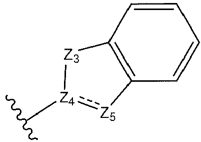 Z₃ is N, O, S or C=O, when the bond between Z₄ and Z₅ is a single bond, Z₄ is N or CH, Z₅ is CH₂ or C=O, when the bond between Z₄ and Z₅ is a double bond, Z₄ is C, Z₅ is CH;
as well as pharmaceutically acceptable salt, solvate, active metabolite, polymorph, ester, optical isomer, prodrug or combinations thereof.

In certain embodiments, compounds disclosed herein have formula (II) or formula (III): 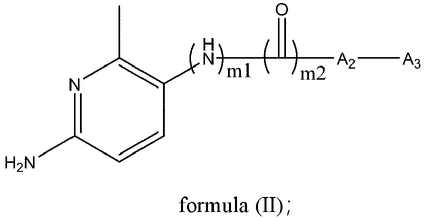 or 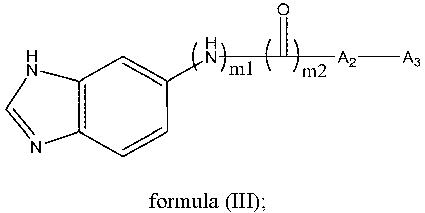 wherein:
A₂ is -CH₂-, -CH=CH-, -C(CH₃)=CH-, -O(CH₂)-, -O(CH₂)₂-, -NH-, -N(CH₃)-, -SO₂-, -NHS(O)₂-, -(CH₂)₂NHS(O)₂- or absent.
A₃ is -CH₃, butenyl, 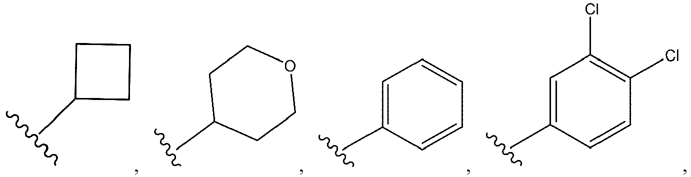 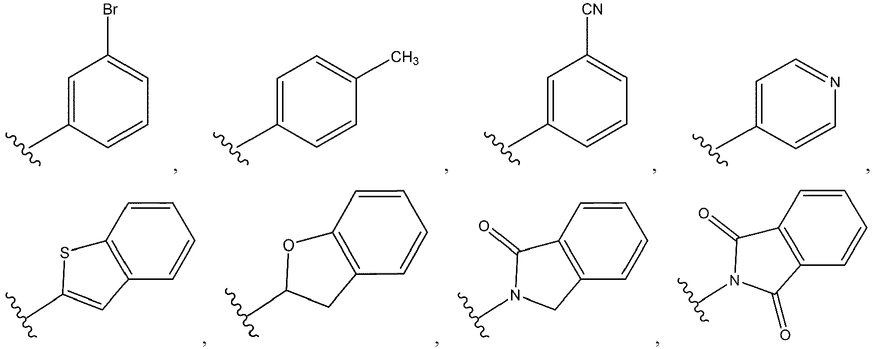 or

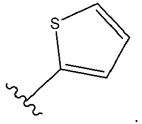

In some embodiments: m1 is 0, and m2 is 1; A₂ is -N(CH₃)-; A₃ is

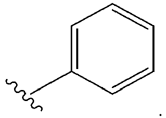

In some embodiments: m1 is 1, and m2 is 0; A₂ is -CH₂-, -SO₂-, -(CH₂)₂NHS(O)₂- or a bond; 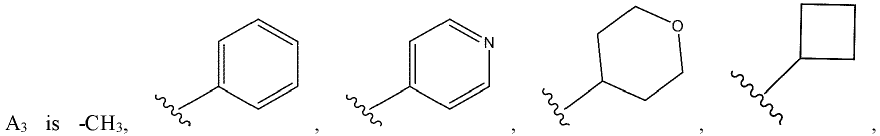 or

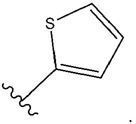

In some embodiments: m1 is 1, and m2 is 1; A₂ is -CH₂-, -NH-, -C(CH₃)=CH- or a bond; A₃ is -CH₃, -C(CH₃)=CH-CH₃, 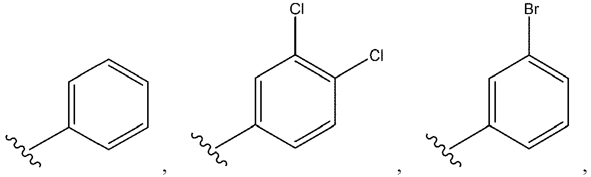 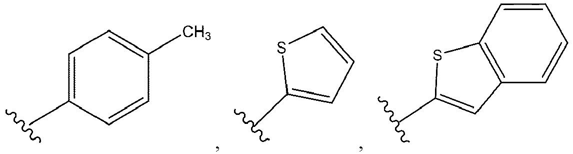 or

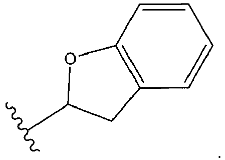

In some embodiments: m1 is 0, and m2 is 0; A₂ is -CH₂-, -CH=CH-, -O(CH₂)-, -O(CH₂)₂- or a bond; A₃ is 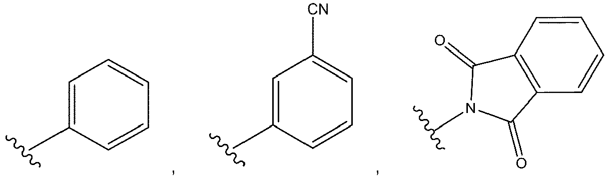 or

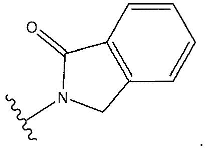

In some embodiments, the compound is:

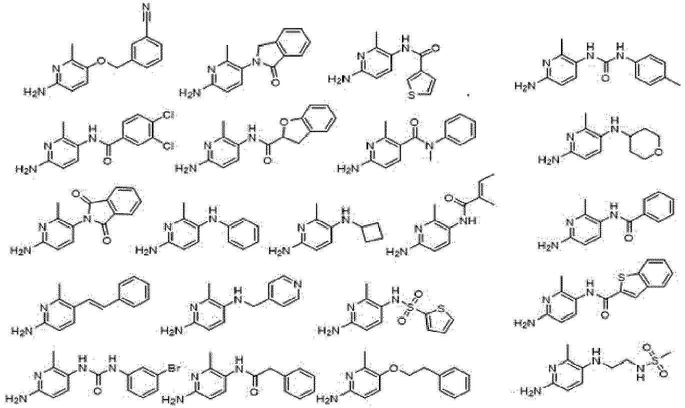

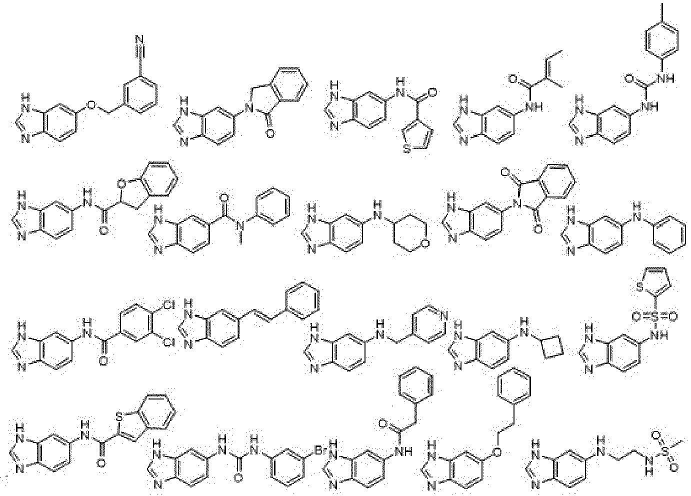

The present invention relates to the pharmaceutical composition, comprising any one of the above compounds and their pharmaceutically acceptable salts, solvates, active metabolites, polymorphs, esters, optical isomers, prodrugs or their combination, and at least one pharmaceutically acceptable carrier or excipient.

The present invention relates to the use of the compound of any one of the statements above and/or its pharmaceutically acceptable salt, solvate, active metabolite, polymorph, ester, optical isomer, prodrug, or combination thereof in preparing drugs of OCT4 high-selectivity activator of induced Pluripotent Stem Cells.

The present invention relates to the application in preparing drugs of OCT4 high-selectivity activator of induced Pluripotent Stem Cells. Diseases treated with high-selectivity OCT4 activator induced Pluripotent Stem Cells include cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, edentulousness, wound healing, bone marrow transplant, osteoarthritis, rheumatoid arthritis, alopecia, blindness, deafness, Crohn's disease and genetic diseases and other similar diseases.

The present invention relates to a method of obtaining induced Pluripotent Stem Cells in subjects with a disease, which comprises administering to the subject a therapeutically effective amount of the compound of any statement above and/or its pharmaceutically acceptable salt, solvate, active metabolite, polymorph, ester, optical isomer, prodrug, or combination thereof.

In the method, the subject with disease refers to Humans who have cancer, heart disease, stroke, diabetes, obesity, Alzheimer's disease, Parkinson's disease, amyotrophic lateral sclerosis, myocardial infarction, muscular nutrition adverse, CMT-1A, spinal cord injury, traumatic brain injury, edentulousness, wound healing, bone marrow transplantation, osteoarthritis, rheumatoid arthritis, alopecia, blindness, deafness, Crohn's disease, genetic disease, and other similar diseases.

The present invention relates to a method for activating the function of OCT4, which comprises making the compound of any statement above and/or its pharmaceutically acceptable salt, solvate, active metabolite, polymorph, ester, optical isomer, prodrug, or combination thereof bind to the OCT4 target protein.

### Detailed Description of the Invention

In the present invention, the following definitions are applicable:
The term "alkyl" herein refers to a straight or branched chain saturated hydrocarbon containing 1-12 carbon atoms. Embodiments of (C1-C6) alkyl include but are not limited to, methyl, ethyl, propyl, butyl, pentyl, hexyl, isopropyl, isobutyl, sec-butyl, tert-butyl, isopentyl, neopentyl and isohexyl.

The term "alkenyl" refers to a straight or branched chain unsaturated hydrocarbon containing 2-12 carbon atoms, containing at least one C=C double bond in the chain. Embodiments of alkenyl groups include vinyl, propenyl, n-butenyl, isobutenyl, pentenyl, or hexenyl.

The term "alkylene": refers to the divalent alkyl group. Any of the monovalent alkyl groups can be an alkylene group by abstracting a second hydrogen atom from the alkyl group. The alkylene group, as defined herein, may also be a C1-C6 alkylene group. The alkylene group may further be the C1-C4 alkylene group. Typical alkylene groups include but are not limited to: -CH₂-, -CH(CH₃)-, -C(CH₃)₂-, -CH₂CH₂-, -CH₂CH(CH₃)-, -CH₂C(CH₃)₂-, -CH₂CH₂CH₂-, -CH₂CH₂CH₂CH₂-, etc.

The term "alkenylene": refers to the divalent alkenyl group. Any of the monovalent alkenyl groups can be an alkenylene by the abstraction of a second hydrogen atom from the alkenyl group. As defined herein, alkenylene may further be C2-C6 alkenylene. Typical alkenylene groups include but are not limited to: -CH=CH-, -CH=C(CH₃)-, -CH=CHCH₂-, -CH=CHCH₂CH₂-, -CH=CHCH₂CH₂CH₂-, -CH=CHCH₂CH₂CH₂CH₂-, etc.

The term "cycloalkyl" refers to a monocyclic saturated carbocyclic ring containing 3-18 carbon atoms. The cycloalkyl group may further be the C4-C6 cycloalkyl group. Embodiments of cycloalkyl include but are not limited to, cyclopropyl, cyclobutyl, cyclopentyl, cyclohexyl, cycloheptyl, cyclooctyl, etc.

The term "halogen" refers to fluorine, chlorine, bromine, or iodine.

The term "cyano group " refers to a substituent having a carbon atom attached to a nitrogen atom by a triple bond (i.e., C≡N).

The term "substitution" as used herein, refers to replacing any one or more hydrogen atoms on a specified atom or group with a group selected from the specified range, provided that the specified atom's normal valence is not exceeded.

The compounds described herein include but are not limited to, their optical isomers, racemates, and other mixtures. In these cases, the individual enantiomers or diastereoisomers, i.e., the optically active configuration, can be obtained by asymmetric synthesis or by resolution of racemate or diastereomer mixtures. Resolution of racemate or diastereomer mixtures can be accomplished by conventional methods such as crystallization in the presence of a resolution agent or chromatography using, for example, chiral High-Performance Liquid Chromatography (HPLC) columns. In addition, these compounds include R- and S-configuration compounds with chiral centers. These compounds also include crystal forms, including polymorphs and clathrate compounds. Similarly, the term "Salt" also includes all isomers, racemates, other mixtures, R- and S-configurations, tautomers, and crystal forms of the salt of said compounds.

"Pharmaceutically acceptable salt" refers to a salt of free acids or bases of the represented compounds in formula (I), formula (II), or formula (III) that is non-toxic, biologically tolerable, or otherwise biologically appropriate for the individual treatment. See generally: S.M. Berge, et al., "Pharmaceutical Salts", J. Pharm. Sci., 1977, 66: 1-19, and Handbook of Pharmaceutical Salts, Properties, Selection, and Use, Stahl and Wermuth, Eds., Wiley-VCH and VHCA, Zurich, 2002. Preferably, pharmaceutically acceptable salts are those that are pharmacologically effective and suitable for contact with patient tissues without undue toxicity, irritation, or anaphylaxis. The compound of formula (I), formula (II), or formula (III) can have enough acidic groups, enough basic groups, or have both these two types of functional groups and correspondingly react with some inorganic or organic bases and inorganic and organic acids to form a pharmaceutically acceptable salt. Examples of pharmaceutically acceptable salts include sulfate, pyrosulfate, bisulfate, sulfite, bisulfite, phosphate, monohydrophosphate, dihydrophosphate, metaphosphate, pyrophosphate, hydrochloride, hydrobromide, hydroiodide, acetate, propionate, decanoate, octanoate, acrylate, formate, isobutyrate, hexanoate, heptanoate, propiolate, oxalate, malonate, succinate, suberate, sebacate, fumarate, maleate, butyne-1,4-dicarboxylate, hexane-1,6-dicarboxylate, benzoate, chlorobenzoate, methylbenzoate, dinitrobenzoate, hydroxybenzoate, methoxybenzoate, phthalate, sulfonate, xylene sulfonate, phenylacetate, phenpropionate, phenylbutyrate, citrate, lactate, gamma-hydroxybutyrate, glycolate, tartrate, methanesulfonate, propanesulfonate, naphthalene-1-sulfonate, naphthalene-2-sulfonate and mandelate.

"Solvates" such as "hydrates" are formed by interacting solvents with compounds. The term "compound" includes solvates of the compound, including hydrates. Likewise, "salt" includes solvates of salts, such as hydrates. Suitable solvates are pharmaceutically acceptable solvates, such as hydrates, including monohydrates and hemihydrates.

"Prodrug" may refer to a precursor of a specified compound that, after administration to a subject, undergoes chemical or physiological processes (e.g., solvolysis, enzymatic decomposition) in vivo or reacts under physiological conditions (e.g., the prodrug is converted into a compound of formula (I) at physiological pH) to obtain the compound. A "pharmaceutically acceptable prodrug" is a prodrug that is non-toxic, biologically tolerated, or otherwise biologically appropriate to administer to the person being treated. Exemplary procedures for selecting and preparing suitable prodrug derivatives are described, for example, in "Design of Prodrugs" by H. Bundgaard, Elsevier, 1985.

"Active metabolite" refers to a pharmaceutically active product of the compound of formula (I), formula (II), or formula (III) or its salts metabolized in vivo. The prodrug and the active metabolites of a compound can be assayed using routine techniques known or available in the art. See, for example, Bertolini et al., J. Med. Chem. 1997, 40, 2011-2016; Shan et al., J. Pharm. Sci. 1997, 86(7), 765-767; 220-230; Bodor, Adv. Drug Res. 1984, 13, 224-331; Bundgaard, Design of Prodrugs (Elsevier Press, 1985); and Larsen, Design and Application of Prodrugs, Drug Design and Development (Krogsgaard-Larsen et al., eds., Harwood Academic Publishers, 1991).

"Therapeutically effective dose" refers to the dose of a compound disclosed herein that, when administered to a mammal (preferably a human), is sufficient for the treatment of a disease or condition (as defined below) of a mammal (preferably a human). The dose of a disclosed compound that constitutes a "therapeutically effective dose" can vary with the compound, the disease and its severity, and the age of the mammal being treated, but can be determined by general skilled personnel in the art based on his own knowledge and the disclosure herein.

The term "treating" refers to administering at least one compound and/or its at least one pharmaceutically acceptable salt described herein to an individual to slow down (reduce) an undesired physiological change or disease, such as the development or spread of inflammation or cancer. The invention objects and the beneficial or desired clinical outcomes include but are not limited to relieving symptoms, reducing disease severity, stabilizing (i.e., not worsening) disease state, delaying or slowing of disease progression, ameliorating or palliating of illness state, and alleviating (whether partial or total) whether detected or undetected disease. "Treatment" also means prolonging survival compared to the expected survival of those not receiving treatment. Individuals needing treatment include those with symptoms of or suffering from these diseases. Pharmaceutical composition

The present invention provides the pharmaceutical composition, which comprises one or more compounds described herein or their pharmaceutically acceptable salts or esters as active ingredients, and one or more pharmaceutically acceptable excipients and carriers, including inert solid diluents and fillers, diluents, including sterile aqueous solutions and various organic solvents, penetration enhancers, solubilizers, and adjuvants. The pharmaceutical composition can be administered alone or with other therapeutic agents. Such compositions are prepared in a manner well-known in the art of pharmacy.

The pharmaceutical composition may be administered in single or multiple doses by any of the acceptable means of pharmaceutical preparations with similar use, such as those described in those patents and patent applications introduced herein by reference, including rectal, buccal, intranasal and transdermal pathways, by intraarterial injection, intravenously, intraperitoneally, parenterally, intramuscularly, subcutaneously, orally, topically, as an inhalant, or by implants or coated devices, such as stents or arterially inserted columnar polymers.

The present invention also provides a kit, comprising a pharmaceutical composition comprising a compound of the present invention or a pharmaceutically acceptable salt thereof, as well as at least one pharmaceutically acceptable carrier.

"Pharmaceutically acceptable carrier or excipient" means a substance that is non-toxic, biologically tolerable, and other biologically appropriate to administer to an individual, such as an inert substance, which is added to a pharmacological composition or as a medium, carrier, or diluted to facilitate administration of the active ingredient and be compatible it. Examples of excipients include calcium carbonate, calcium phosphate, various sugars or starches, cellulose derivatives, gelatin, vegetable oils, and polyethylene glycols.

### Uses of compounds and compositions thereof

The invention provides the application of the compound and its composition, which is mainly used as an OCT4 high-selectivity activator for activating the OCT4 function. Through the chemical regulation of the OCT4 promoter, the expression regulation of its downstream genes is realized, and then pluripotent stem cells can be induced in subjects with diseases to achieve the purpose of disease treatment. The diseases include cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, edentulousness, wound healing, bone marrow transplantation, osteoarthritis, rheumatoid arthritis, alopecia, blindness, deafness, Crohn's disease and genetic diseases and other similar diseases.

List of abbreviations used in the following embodiments and elsewhere herein:
Atm: atmosphere; Boc: t-Butyl oxy carbonyl; (Boc)₂O: di-t-butyl dicarbonate; CH₂Cl₂: dichloromethane; Cs₂CO₃: cesium carbonate; Cu(I)Br: copper bromide; Cu₂SO₄: sulfurous acid copper; DCM: dichloromethane; DMAC: N,N-dimethylacetamide; DMF: N,N-dimethylformamide; DIEA: N,N-diisopropylethylamine; DIOX: 1,4-dioxane; dioxane: dioxane; EA: acetic acid; Et₃N: triethylamine; ETOH: ethanol; EtOAc: ethyl acetate; FA: formic acid; g: gram; h: hour; H₂: hydrogen; HATU: 2-(7-Azabenzotriazol-1-yl)-N,N,N',N'-tetramethyluronium hexafluorophosphat; HBr: hydrobromic acid; (Hbim)BF₄: 1-butylimidazole tetrafluoroborate; H₂O: water; HAc: acetic acid; H₂O₂: hydrogen peroxide; H₂SO₄: oleum; KCN: potassium cyanide; K₂CO₃: potassium carbonate; Li: lithium; MCA: chloroacetic acid; MeCN: acetonitrile; MeOH: methanol; mg: milligram; mL: milliliter; mmol: millimole; mol: mole; m/z: mass-charge ratio; N₂: nitrogen; NaBH₃CN: sodium cyanoborohydride; NaNO₂: sodium nitrite; NaOH: sodium hydroxide; NaOMe: sodium methoxide; Na₂SO₄: sodium sulfate; Ni(OAc)₂4H₂O: nickel acetate; Pd-C: palladium on carbon; PH: power of hydrogen; TBN: tert-buty nitrite; TEA: triethylamine; THF: tetrahydrofuran; TLC: thin-layer chromatography; Xylene: xylene.

Next, in conjunction with the drawings and the present invention embodiments, the technical means adopted by the present invention to achieve the intended purpose of the invention will be further elaborated. The experimental methods in the following embodiments are conventional methods unless otherwise specified. The raw materials, reagent materials, etc., used in the following embodiments are all commercially available products unless otherwise specified.

### General synthesis

The general synthetic routes described in this application can be varied by substituting starting materials with other materials having similar structures, resulting in a different product accordingly. The following synthetic route descriptions give a number of embodiments of how the starting materials can be varied to obtain the corresponding products.

### General Methods A-1-n

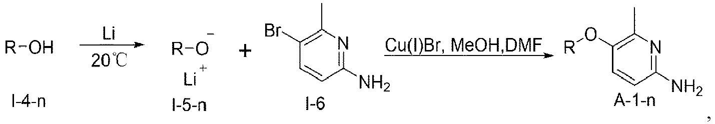

wherein R is 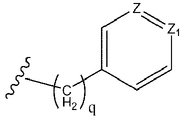 , wherein q is 1 or 2 or 3 or 4, Z and Z₁ are N or CR₂, respectively, and R2 is selected from H, halogen, C1-C4 alkyl or cyano.

### General method A-2-n

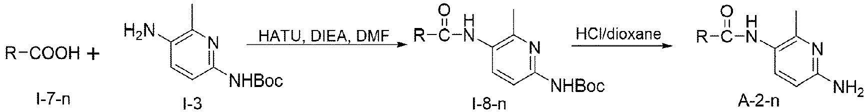

, wherein R is C2-C6 alkenyl; 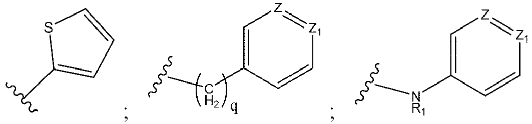 ; or 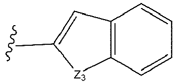 , wherein, q is 1 or 2 or 3 or 4, Z and Z₁ are N or CR₂, respectively, R2 is selected from H, halogen, C1-C4 alkyl or cyano group, R1 is selected from H or C1-C4 alkyl, and Z₃ is N, O, S or C=O.

### General Methods A-4-n

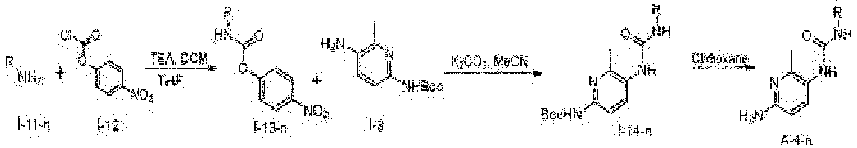

, wherein R is 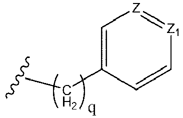 , wherein q is 1 or 2 or 3 or 4, Z and Z₁ are N or CR₂, respectively, and R2 is selected from H, halogen, C1-C4 alkyl or cyano.

### General Methods A-6-n

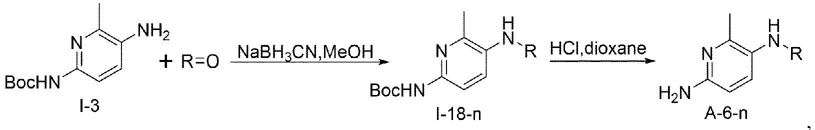

wherein R is C4-C6 cycloalkyl, wherein one carbon atom can be replaced by N, O, S heteroatom; or R=O is 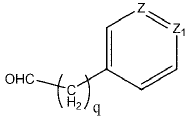 , wherein q is 1 or 2 or 3 or 4, Z and Z₁ are N or CR₂, and R2 is selected from H, halogen, C1-C4 alkyl or cyano.

### General method B-1-n

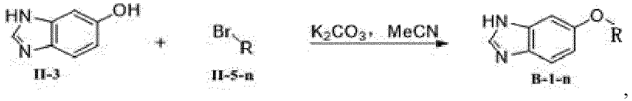

wherein R is as defined in general method A-1-n.

### General method B-3-n

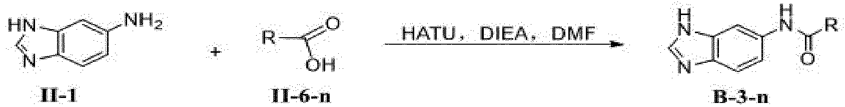

, wherein R is as defined in general method A-2-n.

### General method B-4-n

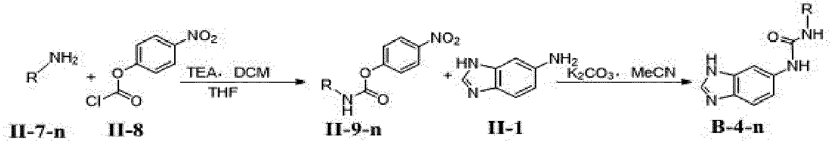

, wherein R is as defined in general method A-4-n.

### General method B-6-n

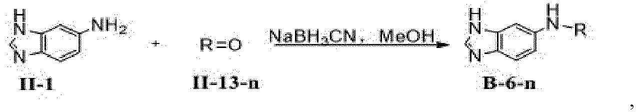

wherein R is as defined in general method A-6-n.

### Intermediate synthesis

### Intermediate 1-3: tert-Butyl (5-amino-6-methylpyridin-2-yl)carbamate

### Synthetic route:

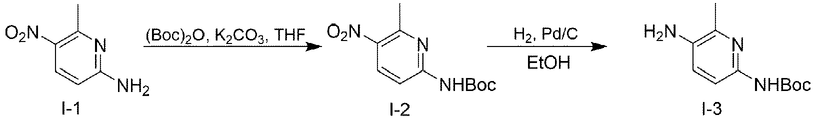

Procedure 1: 6-Methyl-5-nitropyridine-2-amine 1-1 (15.3 g, 0.1 mol) was mixed with di-tert-butyl dicarboxylic anhydride (26.2 g, 0.12 mol) and K₂CO₃ (69 g, 0.5 mol), and tetrahydrofuran (200 mL) was added, which was stirred at room temperature for 3 h. Ethyl acetate and water were added to the reaction mixture, and the organic layer was separated, which was washed with water, dried over sodium sulfate and distilled off the solvent under reduced pressure, obtaining tert-butyl (6-methyl-5-nitropyridin-2-yl)carbamate 1-2 (23.3g, 92%), liquid mass spectrum m/z=254.1[M+H]+.

Procedure 2: The above tert-butyl (6-methyl-5-nitropyridin-2-yl)carbamate 1-2 (12.7 g, 0.05 mol) was stirred with 10% Pd-C (1 g) and MeOH (100 mL) in a hydrogen atmosphere (1 atm) at room temperature for 2 h. The mixture was filtered and distilled off the solvent under reduced pressure, obtaining the intermediate tert-butyl (5-amino-6-methylpyridin-2-yl)carbamate 1-3 (11 g, 98%), liquid phase mass spectrum m/z=224.1[M+H]+.

### Intermediate II-3: 1H-Benzo[d]imidazol-6-ol

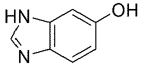

### Synthetic route:

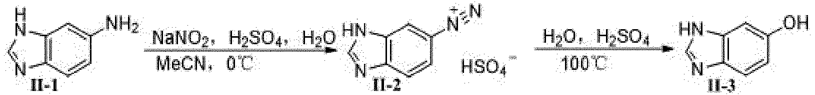

### Procedure 1: Intermediate 1H-benzo[d]imidazol-6-diazo II-2

Water (2 mL) and 20% aqueous H₂SO₄ (1 mL) were added to 1H-benzo[d]imidazol-6-amine (67 mg, 0.5 mmol). Aqueous solution (0.5 mL) of sodium nitrite (42 mg, 0.6 mmol) and acetonitrile (2 mL) were added to the mixture under ice-cooling, and the mixture was stirred for 30 min. Cu₂SO₄ (67 mg, 0.3 mmol) in 20% aqueous HBr (0.5 mL) at room temperature was added to the obtained reaction mixture, and the mixture was stirred at 80°C for 30 min. Ethyl acetate and water were added to the reaction mixture, and the organic layer was separated, which was rinsed with brine, dried over sodium sulfate, and concentrated under reduced pressure. The residue was purified by silica gel column chromatography (chloroform: methanol = 50:1) to obtain II-2 bisulfate (85 mg, 70%), liquid phase mass spectrum m/z = 145.1[m]+.

### Procedure 2: Intermediate 1H-benzo[d]imidazol-6-ol II-3

An aqueous solution (1 mL) of 1H-benzo[d]imidazol-6-diazonium hydrogensulfate (48 mg, 0.2 mmol) was added dropwise to 40% aqueous solution of sulfuric acid (5 mL) at 100°C, and the mixture was stirred for 10 min. NaOH was added to the obtained reaction mixture until the pH was about 3, ethyl acetate was added to the reaction mixture, and the organic layer was separated, which was rinsed with brine, dried over sodium sulfate, and concentrated under reduced pressure. The residue was purified by silica gel column chromatography (chloroform: methanol = 50:1) to obtain II-3 (19 mg, 69%), liquid phase mass spectrum m/z = 134.1[m+H]+.

### Embodiment 1: 3-(((6-Amino-2-methylpyridin-3-yl)oxy)methyl)benzonitrile A-1-1

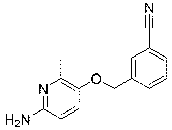

### Procedure 1: Lithium (3-cyanophenyl)methoxide I-5-1

(3-cyanophenyl)methanol (20 mg, 0.15mol) was mixed with lithium metal (12 mg, 1.73 mmol), and tetrahydrofuran (3 mL) was added, which was stirred at 20°C for 3h. The mixture was filtered and distilled off the solvent under reduced pressure, obtaining lithium (3-cyanophenyl)methoxide I-5-1(20 mg, 96%).

### Procedure 2: 3-(((6-Amino-2-methylpyridin-3-yl)oxy)methyl)benzonitrile

Lithium (3-cyanophenyl)methoxide I-5-1 (20 mg, 0.14 mol) was mixed with 5-bromo-6-methylpyridine-2-amine I-6 (32 mg, 0.17 mol), and bromide cuprous (28 mg, 0.2 mol), methanol (1 mL), and N,N dimethylformamide (3 mL) were added, which was stirred in a heated oil bath at 110°C for 1h. Ethyl acetate and water were added to the reaction mixture, and the organic layer was separated, which was dried over sodium sulfate and concentrated under reduced pressure. The residue was purified by silica gel column chromatography (dichloromethane: methanol = 40:1), obtaining 3-(((6-amino-2-methylpyridin-3-yl)oxy)methyl)benzonitrile A-1-1 (26 g, 78%), liquid phase mass spectrum m/z = 240.1[M+H]+.

### Embodiment 2: 6-Methyl-5-phenethoxypyridine-2-amine A-1-2

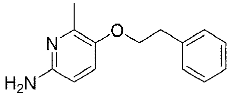

### Procedure 1: Lithium 2-phenylethoxide I-5-2

2-Phenylethanol (18 mg, 0.15mol) was mixed with metal lithium (12 mg, 1.73 mmol), and tetrahydrofuran (3 mL) was added, which was stirred at 20°C for 3h. The mixture was filtered and distilled off the solvent under reduced pressure, obtaining lithium 2-phenylethoxide I-5-2 (18 mg, 94%).

### Procedure 2: 6-Methyl-5-phenethoxypyridine-2-amine A-1-2

Lithium 2-phenylethoxide I-5-2 (18 mg, 0.14 mol) was mixed with 5-bromo-6-methylpyridine-2-amine (32 mg, 0.17 mol), and copper bromide (28 mg, 0.2 mol), methanol (1 mL), and N,N dimethylformamide (3 mL) were added, which was stirred in a heated oil bath at 110°C for 1 h. Ethyl acetate and water were added to the reaction mixture, and the organic layer was separated, which was dried over sodium sulfate and concentrated under reduced pressure. The residue was purified by silica gel column chromatography (dichloromethane: methanol = 40:1), obtaining 6-methyl-5-phenethoxypyridin-2-amine (22 g, 69%), liquid phase mass spectrum m/z = 228.1[M+H]+.

### Embodiment 3: N-(6-Amino-2-methylpyridin-3-yl)thiophene-3-carboxamide A-2-1

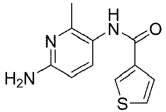

### Procedure 1: tert-Butyl (6-methyl-5-(thiophene-3-carboxamido)pyridin-2-yl)carbamate I-8-1

tert-Butyl (5-amino-6-methylpyridin-2-yl)carbamate (22 mg, 0.1 mmol) was mixed with thiophene-2-carboxylic acid (15 mg, 0.12 mmol) and 2-(7-azabentriazole)-N,N,N',N'-tetramethylurea hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-butyl (6-methyl-5-(thiophene-3-carboxamido)pyridin-2-yl)carbamate I-8-1 (16 mg, 48%), liquid mass spectrum m/z = 334.1[M+H]+.

### Procedure 2: N-(6-Amino-2-methylpyridin-3-yl)thiophene-3-carboxamide

Dioxane solution of HCl (5%, 5 mL) was added to tert-butyl (6-methyl-5-(thiophene-3-carboxamido)pyridin-2-yl)carbamate (16 mg, 0.05 mmol), which was heated and stirred at 70°C for 3h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining N-(6-amino-2-methylpyridin-3-yl)thiophene-3-carboxamide A-2-1 (11 mg, 94%), liquid phase mass spectrum m/z = 234.0[M+H]+.

### Embodiment 4: (E) N-(6-Amino-2-methylpyridin-3-yl)-2-methyl-2-enamide A-2-2

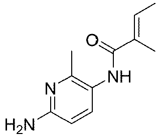

### Procedure 1: tert-Butyl (E)-(6-methyl-5-(2-methyl-2-enamido)pyridin-2-yl)carbamate I-8-2

tert-Butyl (5-amino-6-methylpyridin-2-yl)carbamate (22 mg, 0.1 mmol) was mixed with(E)-2-methyl-2-enoic acid (12 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7), obtaining tert-Butyl (E)-(6-methyl-5-(2-methyl-2-enamido)pyridin-2-yl)carbamate I-8-2 (19 mg, 62%), liquid mass spectrum m/z = 306.1[M+H]+.

### Procedure 2: (E) N-(6-Amino-2-methylpyridin-3-yl)-2-methyl-2-enamide

Dioxane solution of HCl (5%, 5 mL) was added to tert-butyl (E)-(6-methyl-5-(2-methyl-2-enamido)pyridin-2-yl)carbamate (19 mg, 0.06 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining (E) N-(6-amino-2-methylpyridin-3-yl)-2-methyl-2-enamide A-2-2 (12 mg, 97%), liquid mass spectrum m/z = 206.1[M+H]+.

### Embodiment 5: N-(6-Amino-2-methylpyridin-3-yl)-2,3-dihydrobenzofuran-2-carboxamide A-2-3

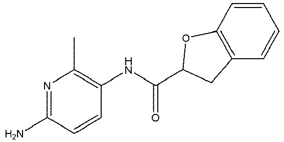

### Procedure 1: tert-Butyl (5-(2,3-dihydrobenzofuran-2-carboxamide)-6-methylpyridin-2-yl)carbamate I-8-3

tert-Butyl (5-amino-6-methylpyridin-2-yl)carbamate (22 mg, 0.1 mmol) was mixed with benzofuran-2-carboxylic acid (19 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-butyl (5-(2,3-dihydrobenzofuran-2-carboxamide)-6-methylpyridin-2-yl)carbamate I-8-3 (20 mg, 55%). liquid mass spectrum m/z = 370.2[M+H]+.

### Procedure 2: N-(6-Amino-2-methylpyridin-3-yl)-2,3-dihydrobenzofuran-2-carboxamide

Dioxane solution of HCl (5%, 5 mL) was added to tert-butyl (5-(2,3-dihydrobenzofuran-2-carboxamide)-6-methylpyridin-2-yl)carbamate (20 mg, 0.055 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining N-(6-amino-2-methylpyridin-3-yl)-2,3-dihydrobenzofuran-2-carboxamide A-2-3 (11 mg, 74%) LC/MS m/z = 270.1[M+H]+.

### Embodiment 6: N-(6-Amino-2-methylpyridin-3-yl)-3,4-dichlorobenzamide A-2-4

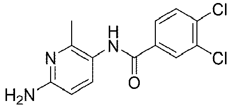

### Procedure 1: tert-Butyl (5-(3,4-dichlorobenzamide)-6-methylpyridin-2-yl)carbamate I-8-4

tert-Butyl (5-amino-6-methylpyridin-2-yl)carbamate (22 mg, 0.1 mmol) was mixed with 3,4-dichlorobenzoic acid (23 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-butyl (5-(3,4-dichlorobenzamide)-6-methylpyridin-2-yl)carbamate I-8-4 (20 mg, 51%), liquid mass spectrum m/z = 396.1 [M+H]+.

### Procedure 2: N-(6-amino-2-methylpyridin-3-yl)-3,4-dichlorobenzamide A-2-4

Dioxane solution of HCl (5%, 5 mL) was added to tert-butyl (5-(3,4-dichlorobenzamide)-6-methylpyridin-2-yl)carbamate (20 mg, 0.05 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain N-(6-amino-2-methylpyridin-3-yl)-3,4-dichlorobenzamide A-2-4 (14 mg, 86%), liquid mass spectrum m/z = 295.0[M+H]+.

### Embodiment 7: N-(6-amino-2-methylpyridin-3-yl) benzothiophene-2-carboxamide A-2-5

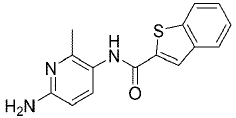

### Procedure 1: tert-Butyl (5-(benzothiophene-2-carboxamide)-6-methylpyridin-2-yl)carbamate I-8-5

tert-Butyl (5-amino-6-methylpyridin-2-yl)carbamate (22 mg, 0.1 mmol) was mixed with benzothiophene-2-carboxylic acid (21 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-butyl (5-(benzothiophene-2-carboxamide)-6-methylpyridin-2-yl)carbamate I-8-5 (22 mg, 57%), liquid mass spectrum m/z = 384.1[M+H].

### Procedure 2: N-(6-Amino-2-methylpyridin-3-yl)benzothiophene-2-carboxamide

Dioxane solution of HCl (5%, 5 mL) was added to tert-butyl (5-(benzothiophene-2-carboxamide)-6-methylpyridin-2-yl)carbamate (22 mg, 0.057 mmol), which was heated and stirred at 70°C for 3h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain N-(6-amino-2-methylpyridin-3-yl) benzothiophene-2- carboxamide A-2-5 (11 mg, 68%), liquid mass spectrum m/z = 284.1 [M+H]+.

### Embodiment 8: N-(6-amino-2-methylpyridin-3-yl)-2-phenylacetamide A-2-6

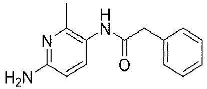

### Procedure 1: tert-Butyl (6-methyl-5-(2-phenylacetamide)pyridin-2-yl)carbamate I-8-6

tert-Butyl (5-amino-6-methylpyridin-2-yl)carbamate (22 mg, 0.1 mmol) was mixed with 2-phenylacetic acid (16 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-Butyl (6-methyl-5-(2-phenylacetamide)pyridin-2-yl)carbamate I-8-6 (18 mg, 53%), liquid phase mass spectrum m/z = 342.2[M+H]+.

### Procedure 2: N-(6-Amino-2-methylpyridin-3-yl)-2-phenylacetamide:

Dioxane solution of HCl (5%, 5 mL) was added to tert-butyl (6-methyl-5-(2-phenylacetamide) pyridin-2-yl)carbamate (18 mg, 0.05 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining N-(6-amino-2-methylpyridin-3-yl)-2-phenylacetamide A-2-6 (12 mg, 94%), liquid mass spectrum m/z = 242.1[M+H]+.

### Embodiment 9: N-(6-amino-2-methylpyridin-3-yl)benzamide A-2-7

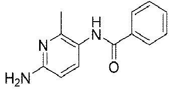

### Procedure 1: Intermediate tert-Butyl (5-benzamido-6-methylpyridin-2-yl)carbamate 1-8-7

tert-Butyl (5-amino-6-methylpyridin-2-yl)carbamate (22 mg, 0.1 mmol) was mixed with 2-benzoic acid (15 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) was added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain tert-butyl (5-benzamido-6-methylpyridin-2-yl)carbamate 1-8-7 (15 mg, 46%), liquid phase mass spectrum m/z = 328.2[M+H]+.

### Procedure 2: N-(6-Amino-2-methylpyridin-3-yl)benzamide

Dioxane solution of HCl (5%, 5 mL) was added to tert-butyl (5-benzamido-6-methylpyridin-2-yl)carbamate (15 mg, 0.046 mmol), which was heated and stirred at 70°C 3h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining N-(6-amino-2-methylpyridin-3-yl) benzamide A-2-7 (10 mg, 96%), liquid mass spectrum m/z = 227.1[M+H]+.

### Embodiment 10: 2-(6-Amino-2-methylpyridin-3-yl) isoindol-1- one A-3

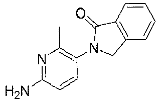

Its synthetic route is:

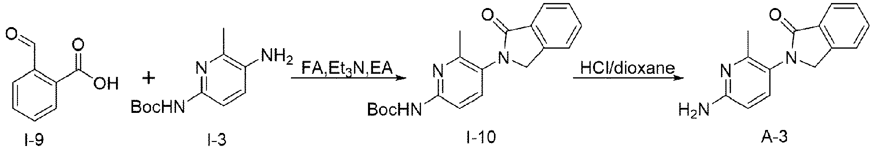

### Procedure 1: tert-Butyl (6-methyl-5-(1-oxoisoindol-2-yl)pyridin-2-yl)carbamate I-10

Tert-butyl (5-amino-6-methylpyridin-2-yl)carbamate (22 mg, 0.1 mmol) was mixed with 2-formylbenzoic acid (18 mg, 0.12 mmol). Formic acid (0.2 mL), three ethylamine (1 mL), and ethanol (1 mL) were added, which was heated to 80°C and stirred for 60 min. Ethyl acetate and water were added to the reaction mixture, and the organic layer was separated, which was dried over sodium sulfate and concentrated under reduced pressure. The residue was purified by silica gel column chromatography (dichloromethane: ethyl acetate = 20:1) to obtain tert-butyl (6-methyl-5-(1-oxoisoindol-2-yl)pyridin-2-yl)carbamate I-10 (20 mg, 59%), liquid mass spectrum m/z = 340.4[M+H]+.

### Procedure 2: 2-(6-Amino-2-methylpyridin-3-yl)isoindol-1-one

Dioxane solution of HCl (5%, 5 mL) was added to tert-butyl(6-methyl-5-(1-oxoisoindol-2-yl) pyridin-2-yl)carbamate (20 mg, 0.059 mmol). The mixture was heated and stirred at 70°C for 3 h. Ethyl acetate and water was added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain 2-(6-amino-2-methylpyridin-3-yl) isoindol-1-one A-3 (12 mg, 85%), liquid mass spectrum m/z = 240.1[M+H]+.

### Embodiment 11: 1-(6-amino-2-methylpyridin-3-yl)-3-(p-tolyl)urea A-4-1

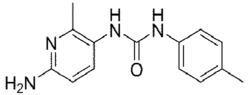

### Procedure 1: p-nitrophenyl p-toluidine carbonate I-13-1

P-toluidine (21 mg, 0.2 mmol) was mixed with 4-nitrophenylcarbonyl chloride (48 mg, 0.24 mmol), and triethanolamine (0.2 mL), dichloromethane (1 mL), and tetrahydrofuran (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (dichloromethane: hexane = 5: 1) to obtain p-nitrophenyl p-toluidine carbonate I-13-1 (42 mg, 77%), liquid phase mass spectrum m/z = 273.1[M+H]+.

### Procedure 2: tert-Butyl (6-methyl-5-(3-(p-tolyl)ureido)pyridin-2-yl)carbamate I-14-1

p-Nitrophenyl p-toluene carbamate (27 mg, 0.1 mmol) was mixed with tert-butyl (5-amino-6-methylpyridin-2-yl)carbamate (26 mg, 0.12 mmol) and K₂CO₃ (0.2 g, 1.45 mmol), and acetonitrile (3 mL) was added, which was heated to 40°C and stirred for 4 h. After completion of the reaction (monitored by TLC), the reaction mixture was separated and concentrated in vacuo. The crude reaction mixture thus obtained was washed with dichloromethane, then EtOAc and finally MeOH (1 mL each). Finally, the reaction product was recrystallized using EtOAc (under warm conditions) to obtain pure tert-butyl (6-methyl-5-(3-(p-tolyl)ureido)pyridin-2-yl)carbamate I-14-1 (28 mg, 80%), liquid mass spectrum m/z = 357.2[M+H]+.

### Procedure 3: 1-(6-amino-2-methylpyridine-3-yl)-3-(p-tolyl)urea:

Dioxane solution of HCl (5%, 5 mL) was added to tert-butyl (6-methyl-5-(3-(p-tolyl)ureido)pyridin-2-yl)carbamate (28 mg, 0.08 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain urea, 1-(6-amino-2-methylpyridin-3-yl)-3-(p-tolyl)- A-4-1 (16 mg, 78%), liquid phase mass spectrum m/z = 257.1[M+H]+.

### Embodiment 12: 1-(6-amino-2-methylpyridine-3-yl)-3-(p-tolyl)urea A-4-2

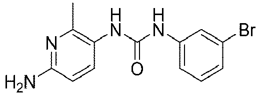

### Procedure 1: 4-Nitrophenyl(3-bromophenyl)aminocarbonate I-13-2

3-Bromoaniline (34 mg, 0.2 mmol) was mixed with 4-nitrophenyl carbonyl chloride (48 mg, 0.24 mmol), and triethanolamine (0.2 mL), dichloromethane (1 mL) and tetrahydrofuran (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (dichloromethane: hexane = 5: 1) to obtain 4-nitrophenyl (3-bromophenyl)aminocarbonate I-13-2 (46 mg, 68%), liquid phase mass spectrum m/z = 338.1[M+H]+.

### Procedure 2: tert-Butyl (5-(3-(3-bromophenyl)ureido)-6-methylpyridin-2-yl)carbamate I-14-2

4-Nitrophenyl (3-bromophenyl)aminocarbonate (34 mg, 0.1 mmol) was mixed with tert-butyl (5-amino-6-methylpyridin-2-yl)carbamate (26 mg, 0.12 mmol) and K₂CO₃ (0.2 g, 1.45 mmol), and acetonitrile (3 mL) was added, which was heated to 40°C and stirred for 4 h. After completion of the reaction (monitored by TLC), the reaction mixture was separated and concentrated in vacuo. The crude reaction mixture thus obtained was washed with dichloromethane, then EtOAc and finally MeOH (1 mL each). Finally, the reaction product was recrystallized using EtOAc (under warm conditions) to obtain a pure form of tert-butyl (5-(3-(3-bromophenyl)ureido)-6-methylpyridin-2-yl)carbamate I-14-2 (34 mg, 81%), liquid mass spectrum m/z = 421.1[M+H]+.

### Procedure 3: 1-(6-amino-2-methylpyridine-3-yl)-3-(p-tolyl)urea

Dioxane solution of HCl (5 %, 5 mL) was added to tert-butyl (5-(3-(3-bromophenyl)ureido)-6-methylpyridin-2-yl)carbamate (34 mg, 0.08 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain urea, 1-(6-amino-2-methylpyridin-3-yl)-3-(p-tolyl)- A-4-2 (20 mg, 78%), liquid phase mass spectrum m/z = 321.0[M+H]+.

### Embodiment 13: 6-Amino-N,2-dimethyl-N-phenylnicotinamide A-5

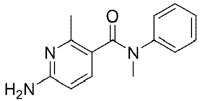

Its synthetic route is:

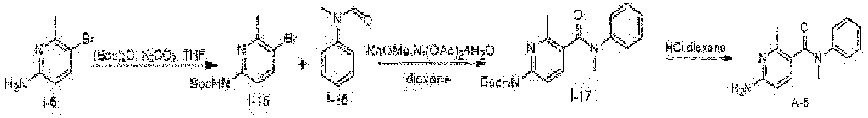

### Procedure 1: tert-Butyl (5-bromo-6-methylpyridin-2-yl)carbamate I-15

5-Bromo-6-methylpyridine-2-amine (37 mg, 0.2 mmol) was mixed with di-tert-butyldicarbonic anhydride (52 mg, 0.24 mmol) and K₂CO₃ (69 mg, 0.5 mmol), and tetrahydrofuran (5 mL) was added, which was stirred at room temperature for 3 h. Ethyl acetate and water were added to the reaction mixture, and the organic layer was separated, which was washed with water, dried over sodium sulfate and distilled off the solvent under reduced pressure, obtaining tert-butyl (5-bromo-6-methylpyridin-2-yl)carbamate I-15 (50 mg, 88%), liquid mass spectrum m/z = 287.0[M+H]+.

### Procedure 2: tert-Butyl (6-methyl-5-(methyl(phenyl)carbamoyl)pyridin-2-yl)carbamate I-17

tert-Butyl (5-bromo-6-methylpyridin-2-yl)carbamate (50 mg, 0.175 mmol) was mixed with N-methyl-N-phenylformamide (27 mg, 0.175 mmol), sodium methoxide (11 mg, 0.2 mmol), and nickel diacetate tetrahydrate (25 mg, 0.1 mmol), and 1,4-dioxane (8 mL) was added to it, which was heated and stirred at 110°C in an oil bath under the helium protection for 12 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain tert-butyl (6-methyl-5-(methyl(phenyl)carbamoyl)pyridin-2-yl)carbamate I-17 (50 mg, 84%), liquid mass spectrum m/z = 342.2[M+H]+.

### Procedure 3: 6-amino-N,2-dimethyl-N-phenylnicotinamide:

Dioxane solution of HCl (5%, 5 mL) was added to tert-butyl (6-methyl-5-(methyl(phenyl)carbamoyl)pyridin-2-yl)carbamate (50 mg, 0.147 mmol), which was heated and stirred at 70°C for 3 h. Ehyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain 6-amino-N,2-dimethyl-N-phenylnicotinamide A-5 (27 mg, 76%), liquid phase mass spectrum m/z = 242.1[M+H]+.

### Embodiment 14: 6-methyl-N⁵-(tetrahydro-2H-pyran-4-yl)pyridine-2,5-diamine A-6-1

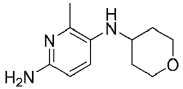

### Procedure 1: tert-Butyl (6-methyl-5-((tetrahydro-2H-pyran-4-yl)amino)pyridin-2-yl)carbamate I-18-1

tert-Butyl (5-amino-6-methylpyridin-2-yl)carbamate (22 mg, 0.1 mmol) was mixed with tetrahydro-4H-pyran-4-one (12 mg, 0.12 mmol), and methanol (2 mL) was added, which was stirred at room temperature for 2 h. Then sodium cyanoborohydride (20 mg, 0.3 mmol) was added and the mixture was stirred at room temperature for 2 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain tert-butyl (6-methyl-5-((tetrahydro-2H-pyran-4- yl)amino)pyridin-2-yl)carbamate I-18-1 (23 mg, 75%), liquid mass spectrum m/z = 308.2[M+H]+.

### Procedure 2: 6-Methyl-N⁵-(tetrahydro-2H-pyran-4-yl)pyridine-2,5-diamine

Dioxygen solution of HCl (5%, 5 mL) was added to tert-butyl(6-methyl-5-((tetrahydro-2H-pyran-4-yl)amino)pyridin-2-yl)carbamate (23 mg, 0.075 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain 6-methyl-N⁵-(tetrahydro-2H-pyran-4-yl)pyridine-2,5-diamine A-6-1 (12 mg, 78%), liquid mass spectrum m/z = 208.1 [M+H]+.

### Embodiment 15: 6-Methyl-N⁵-(pyridin-4-ylmethyl)pyridine-2,5-diamine A-6-2

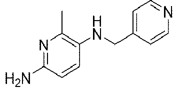

### Procedure 1: tert-Butyl (6-methyl-5-((pyridin-4-ylmethyl)amino)pyridin-2-yl)carbamate I-18-2

Tert-butyl (5-amino-6-methylpyridin-2-yl)carbamate (22 mg, 0.1 mmol) was mixed with isonicotinaldehyde (13 mg, 0.12 mmol), and methanol (2 mL) was added, which was stirred at room temperature for 2 h. Then sodium cyanoborohydride (20 mg, 0.3 mmol) was added, and the mixture was stirred at room temperature for 2 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure, obtaining tert-butyl (6-methyl-5-((pyridin-4-ylmethyl)amino)pyridin-2-yl)carbamate I-18-2 (24 mg, 76%), liquid mass spectrum m/z = 314.2[M+H]+.

### Procedure 2: 6-Methyl-N⁵-(pyridin-4-ylmethyl)pyridine-2,5-diamine:

HCl dioxane solution (5%, 5 mL) was added to tert-butyl(6-methyl-5-((tetrahydro-2H-pyran-4-yl)amino)pyridin-2-yl)carbamate (23 mg, 0.075 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure, obtaining 6-Methyl-N⁵-(pyridin-4-ylmethyl)pyridine-2,5-diamine A-6-2 (12 mg, 78%), liquid mass spectrum m/z = 208.1[M+H]+.

### Embodiment 16: N⁵-cyclobutyl-6-methylpyridine-2,5-diamine A-6-3

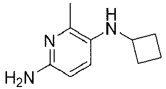

### Procedure 1: tert-Butyl (5-(cyclobutylamino)-6-methylpyridin-2-yl)carbamate I-18-3

tert-Butyl (5-amino-6-methylpyridin-2-yl)carbamate (22 mg, 0.1 mmol) was mixed with cyclobutanone (8 mg, 0.12 mmol), and methanol was added (2 mL), which was stirred at room temperature for 2 h. Then sodium cyanoborohydride (20 mg, 0.3 mmol) was added and stirred at room temperature for 2 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure, obtaining tert butyl (5-(cyclobutylamino)-6-methylpyridin-2-yl)carbamate I-18-3 (20 mg, 72%), liquid mass spectrum m/z = 278.2[M+H]+.

### Procedure 2: N⁵-cyclobutyl-6-methylpyridine-2,5-diamine:

Dioxane solution of HCl (5%, 5 mL) was added to tert-butyl (5-(cyclobutylamino)-6-methylpyridin-2-yl)carbamate (20 mg, 0.072 mmol), which was heated and at stirred 70°C for 3 h. Ethyl acetate and water were added, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain N⁵-cyclobutyl-6-methylpyridine-2,5-diamine A-6-3 (10 mg, 78%), liquid mass spectrum m/z = 178.1[M+H]+.

### Embodiment 17: 2-(6-Amino-2-methylpyridin-3-yl)isoindol-1,3-dione A-7

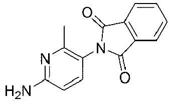

Its synthetic route is:

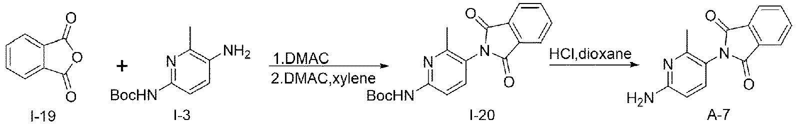

### Procedure 1: tert-Butyl (5-(1,3-dioxoisooctanol-2-yl)-6-methylpyridin-2-yl)carbamate I-20

tert-Butyl (5-amino-6-methylpyridin-2-yl)carbamate (22 mg, 0.1 mmol) was mixed with isobenzofuran-1,3-dione (18 mg, 0.12 mmol), and N,N-dimethylacetamide (2 mL) was added, which was stirred at room temperature for 24 h. Then xylene (1 mL) was added and the mixture was stirred in an oil bath at 140°C for 48 h. After completion (monitored by TLC), the insoluble catalyst was isolated by filtration, washed with acetone and dried. The organic layer was concentrated under reduced pressure to obtain the desired product, which was washed with water and recrystallized in ethanol. The crude product was purified by silica gel column chromatography (CH₂Cl₂/n-hexane = 1:1) and distilled off the solvent under reduced pressure to obtain tert-butyl (5-(1,3-dioxoisooctanol-2-yl)-6-methylpyridin-2-yl)carbamate I-20 (24 mg, 68%) LC/MS m/z = 354.1[M+H]+.

### Procedure 2: 2-(6-Amino-2-methylpyridin-3-yl)isoindol-1,3-dione

Dioxane solution of HCl (5%, 5 mL) was added to tert-butyl (5-(1,3-dioxoisooctanol-2-yl)-6-methylpyridin-2-yl)carbamate (24 mg, 0.068 mmol), which was heated and stirred at 70°C for 3 h. Ethyl acetate and water were added, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain 2-(6-amino-2-methylpyridin-3-yl)isoindol-1,3-ditone A-7 (12 mg, 70%), liquid phase mass spectrum m/z = 254.1 [M+H]+.

### Embodiment 18: 6-Methyl-N⁵-phenylpyridine-2,5-diamine A-8

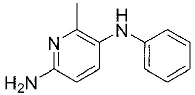

Its synthetic route is:

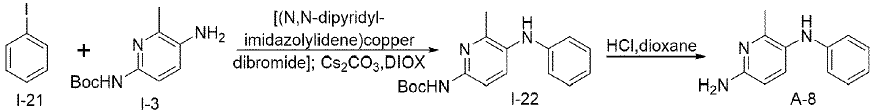

### Procedure 1: tert-Butyl (6-methyl-5-(anilino)pyridin-2-yl)carbamate I-22

tert-Butyl (5-amino-6-methylpyridin-2-yl)carbamate (22 mg, 0.1 mmol) was mixed with iodobenzene (24 mg, 0.12 mmol), (N,N-bipyridyl imidazolylene) copper dibromid (10 mg, 0.023 mmol), and cesium carbonate (100 mg, 0.3 mmol) and 1,4-dioxane (5 mL) was added, which was stirred in an oil bath at 170°C for 12 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain tert-butyl (6-(methyl-5-(anilino)pyridin-2-yl)carbamate I-22 (25 mg, 84%) LC/MS m/z = 300.2[M+H]+.

### Procedure 2: 6-Methyl-N⁵-phenylpyridine-2,5-diamine:

Dioxane solution of HCl (5%, 5 mL) was added to tert-butyl (6-(methyl-5-(anilino) pyridin-2-yl)carbamate (25 mg, 0.084 mmol), which was heated and stirred at 70°C for 3 h. Ethyl acetate and water were added to the reaction mixture, and the organic layer was separated, which was dried over sodium sulfate and concentrated under reduced pressure. The residue was subjected to silica gel column chromatography (ethyl acetate: hexane = 3:7), and distilled off the solvent under reduced pressure to obtain 6-methyl-N⁵-phenylpyridine-2,5-diamine A-8 (12 mg, 72%), liquid phase mass spectrum m/z = 200.1[M+H]+.

### Embodiment 19: (E)-6-methyl-5-styrylpyridine-2-amine A-9

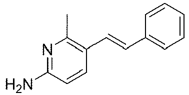

Its synthetic route is:

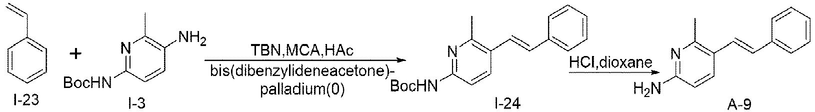

### Procedure 1: tert-Butyl (E)-(6-methyl-5-styrylpyridin-2-yl)carbamate I-24

tert-Butyl (5-amino-6-methylpyridin-2-yl)carbamate (22 mg, 0.1 mmol) was mixed with styrene (13 mg, 0.12 mmol) and bis(dibenzylideneacetone)palladium(0) (5 mg, 0.0087 mmol), which was stirred in an oil bath at 50°C for 2 h after adding nitroso tert-butyl ester (0.5 mL), chloroacetic acid (0.5 mL), and acetic acid (3 mL). Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain tert-butyl (E)-(6-methyl-5-styrylpyridin-2-yl)carbamate I-24 (22 mg, 71%), liquid mass spectrum m/z = 311.2[M+H]+.

### Procedure 2: (E)-6-Methyl-5-styrylpyridine-2-amine

Dioxane solution of HCl (5%, 5 mL) was added to tert-butyl(E)-(6-methyl-5-styrylpyridin-2-yl)carbamate (22 mg, 0.071 mmol), which was heated and stirred at 70°C for 3h. Ethyl acetate and water were added to the reaction mixture, which was dried over sodium sulfate and the organic layer was separated and concentrated under reduced pressure. The residue was subjected to silica gel column chromatography (ethyl acetate: hexane = 3:7) and distilled off the solvent under reduced pressure to obtain (E)-6-methyl-5-styrylpyridine-2-amine A-9 (13 mg, 88%), liquid phase mass spectrum m/z = 211.1 [M+H]+.

### Embodiment 20: N-(6-Amino-2-methylpyridin-3-yl)thiophene-2-sulfonamide A-10

Its synthetic route is:

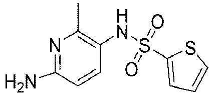

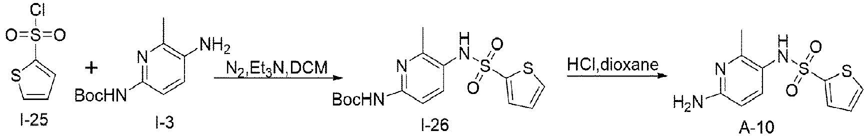

### Procedure 1: tert-Butyl (6-methyl-5-(thiophene-2-sulfonylamino)pyridin-2-yl)carbamate I-26

tert-Butyl (5-amino-6-methylpyridin-2-yl)carbamate (22 mg, 0.1 mmol) was mixed with thiophene-2-sulfonyl chloride (22 mg, 0.12 mmol), and triethylamine (0.5 mL) and dichloromethane (3 mL) were added, which was stirred at room temperature for 24 h under nitrogen protection. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) and distilled off the solvent under reduced pressure to obtain tert-butyl (6-methyl-5-(thiophene-2-sulfonylamino)pyridin-2-yl)carbamate I-26 (19 mg, 52%), liquid mass spectrum m/z = 370.1[M+H]+.

### Procedure 2: N-(6-Amino-2-methylpyridin-3-yl)thiophene-2-sulfonamide

Dioxane solution of HCl (5%, 5 mL) was added to tert-butyl (6-methyl-5-(thiophene-2-sulfonyl)pyridin-2-yl)carbamate (19 mg, 0.052 mmol), which was heated and stirred at 70°C for 3 h. Ethyl acetate and water was added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain N-(6-amino-2-methylpyridin-3-yl)thiophene-2-sulfonamide A-10 (10 mg, 72%), liquid phase mass spectrum m/z = 270.0[M+H]+.

### Embodiment 21: N-(2-((6-amino-2-methylpyridin-3-yl)amino)ethyl)methanesulfonamide A-11

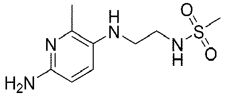

Its synthetic route is:

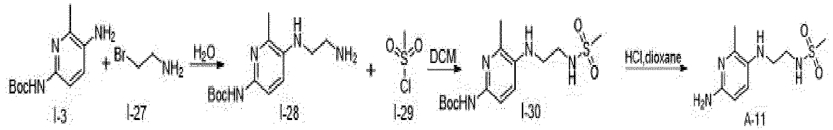

### Procedure 1: tert-Butyl (5-((2-aminoethyl)amino)-6-methylpyridin-2-yl)carbamate I-28

tert-Butyl (5-amino-6-methylpyridin-2-yl)carbamate (44 mg, 0.2 mmol) was mixed with 2-bromoethane-1-amine (29 mg, 0.24 mmol), which was added water (5 mL) and stirred in an oil bath at 95°C for 18 h. Ethyl acetate and water were added to the reaction mixture, and the organic layer was separated, which was dried over sodium sulfate and concentrated under reduced pressure. The mixture was purified by flash chromatography on silica gel (CH₂Cl₂methanol-ammonia) to give the desired product. The solvent was distilled off under reduced pressure to obtain tert-butyl (5-((2-aminoethyl)amino)-6-methylpyridin-2-yl)carbamate I-28 (38 mg, 72%), liquid phase mass spectrum m/ z = 267.2[M+H]+.

### Procedure 2: tert-Butyl (6-methyl-5-((2-(methylsulfonyl)ethyl)amino)pyridin-2-yl)carbamate 1-30

tert-Butyl (5-((2-aminoethyl)amino)-6-methylpyridin-2-yl)carbamate (38 mg, 0.144 mmol) was mixed with methanesulfonyl chloride (28 mg, 0.16 mmol), and dichloromethane (5 mL) was added, which was stirred in an ice-water bath for 24 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) and distilled off the solvent under reduced pressure to obtain tert-butyl (6-methyl-5-((2-(methylsulfonyl)ethyl)amino)pyridin-2-yl)carbamate I-30 (20 mg, 40%), liquid mass spectrum m/z = 255.1[M+H]+.

### Procedure 3: N-(2-((6-amino-2-methylpyridin-3-yl)amino)ethyl)methanesulfonamide

Dioxane solution of HCl (5%, 5 mL) was added to tert-butyl (6-methyl-5-((2-(methylsulfonyl) ethyl)amino)pyridin-2-yl)carbamate (20 mg, 0.057 mmol) , which was heated and stirred at 70°C for 3 h. Ethyl acetate and water was added to the reaction mixture, and the organic layer was separated, which was dried over sodium sulfate and concentrated under reduced pressure. The residue was subjected to silica gel column chromatography (ethyl acetate: hexane = 3:7) and distilled off the solvent under reduced pressure to obtain N-(2-((6-amino-2-methylpyridin-3-yl) amino)ethyl) methane sulfonamide A-11 (11 mg, 78%), liquid phase mass spectrum m/z = 245.1[M+H]+.

### Embodiment 22: 3-((1H-Benzo[d]imidazol-6-yl)oxymethyl)benzonitrile B-1-1

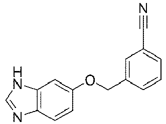

Procedure: 1H-Benzo[d]imidazol-6-ol II-3 (9 mg, 0.07 mmol) was mixed with 3-(bromomethyl) benzonitrile (20 mg, 0.1 mmol) and K₂CO₃ (138 mg, 1 mmol), and acetonitrile (2 mL) was added, which was heated to 40°C and stirred for 10 min. Ethyl acetate and water were added to the reaction mixture, and the organic layer was separated, which was dried over sodium sulfate and concentrated under reduced pressure. The residue was purified by silica gel column chromatography (dichloromethane: methanol = 40:1) to obtain 3-((1H-benzo[d]imidazol-6-yl)oxymethyl)benzonitrile B-1-1 (10 mg, 57%), liquid phase mass spectrum m/z = 249.3 [m+H]+.

### Embodiment 23: 6-phenoxy-1H-benzo[d]imidazole B-1-2

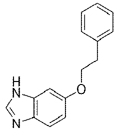

Procedure: 1H-benzo[d]imidazol-6-ol II-3 (9 mg, 0.07 mmol) was mixed with (2-bromoethyl) benzene (22 mg, 0.12 mmol) and K₂CO₃ (138 mg, 1 mmol), and acetonitrile (2 mL) was added, which was heated to 40°C and stirred for 10 min. Ethyl acetate and water were added to the reaction mixture, and the organic layer was separated, which was dried over sodium sulfate and concentrated under reduced pressure. The residue was purified by silica gel column chromatography (dichloromethane: methanol = 40:1) to obtain 6-phenoxy-1H-benzo[d]imidazole B-1-2 (12 mg, 70%), liquid phase mass spectrum m/z = 238.1[m+H]+.

### Embodiment 24: 2-(1H-Benzo[d]imidazol-6-yl)isoindol-1-one B-2

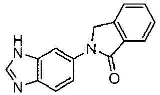

Its synthetic route:

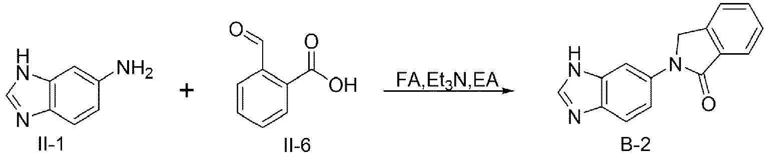

Procedure: 1H-Benzo[d]imidazol-6-amine (13 mg, 0.1 mmol) was mixed with 2-formylbenzoic acid (18 mg, 0.12 mmol), and formic acid (0.2 mL), triethylamine (1 mL), and Ethanol (1 mL) were added, which was heated to 80°C and stirred for 60 min. Ethyl acetate and water were added to the reaction mixture, and the organic layer was separated, which was dried over sodium sulfate and concentrated under reduced pressure. The residue was purified by silica gel column chromatography (dichloromethane: ethyl acetate = 20:1) to obtain 2-(1H-benzo[d]imidazol-6-yl)isoindol-1-one B-2 (13 mg, 54%), liquid phase mass spectrum m/z = 249.1[m+H]+.

### Embodiment 25: N-(1H-benzo[d]imidazol-6-yl)thiophene-3-carboxamide B-3-1

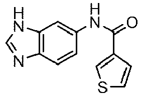

Procedure: 1H-benzo[d]imidazol-6-amine (13 mg, 0.1 mmol) was mixed with thiophene-2-carboxylic acid (15 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain N-(1H-benzo[d]imidazol-6-yl)thiophene-3-carboxamide B-3-1 (9 mg, 37%), liquid phase mass spectrum m/z = 243.1 [m+H]+.

### Embodiment 26: (E)-N-(1H-benzo[d]imidazol-6-yl)-2-methyl-2-enamide B-3-2

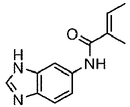

Procedure: 1H-benzo[d]imidazol-6-amine (13 mg, 0.1 mmol) was mixed with (E)-2-methyl-2-enoic acid (12 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) was added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain (E)-N-(1H-benzo[d]imidazol-6-yl)-2-methyl-2-enamide B-3-2 (10 mg, 46%), liquid mass spectrum m/z = 215.1[m+H]+.

### Embodiment 27: N-(1H-benzo[d]imidazol-6-yl)-2,3-dihydrobenzofuran-2-carboxamide B-3-3

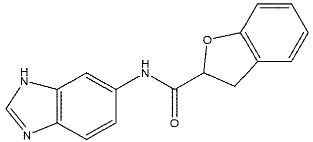

Procedure: 1H-benzo[d]imidazol-6-amine (13 mg, 0.1 mmol) was mixed with benzofuran-2-carboxylic acid (19 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethyl formamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain N-(1H-benzo[d]imidazol-6-yl)-2,3-dihydrobenzofuran-2-carboxamide B-3-3 (12 mg, 41%), liquid mass spectrum m/z = 279.1 [m+H]+.

### Embodiment 28: N-(1H-benzo[d]imidazol-6-yl)-3,4-dichlorobenzamide B-3-4

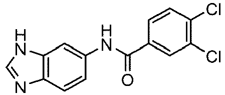

Procedure: 1H-benzo[d]imidazol-6-amine (13 mg, 0.1 mmol) was mixed with 3,4-dichlorobenzoic acid (23 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethyl formamide (2 mL) were added, and which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain N-(1H-benzo[d]imidazol-6-yl)-3,4-dichlorobenzamide B-3-4 (12 mg, 39%), liquid mass spectrum m/z = 305.0[m+H]+.

### Embodiment 29: N-(1H-Benzo[d]imidazol-6-yl)benzo[b]thiophene-2-carboxamide B-3-5

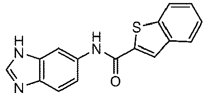

Procedure: 1H-benzo[d]imidazol-6-amine (13 mg, 0.1 mmol) was mixed with benzothiophene-2-carboxylic acid (21 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethyl formamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain N-(1H-benzo[d]imidazol-6-yl)benzo[b]thiophene-2-carboxamide B-3-5 (13 mg, 43%), liquid phase mass spectrum m/z = 293.1[m+H]+.

### Embodiment 30: N-(1H-Benzo[d]imidazol-6-yl)-2-phenylacetamide B-3-6

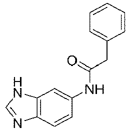

Procedure: 1H-benzo[d]imidazol-6-amine (13 mg, 0.1 mmol) was mixed with 2-phenylacetic acid (16 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) to obtain N-(1H-benzo[d]imidazol-6-yl)-2-phenylacetamide B-3-6 (11 mg, 43%), liquid phase mass spectrum m/z = 251.11[m+H]+.

### Embodiment 31: 1-(1H-benzo[d]imidazol-6-yl)-3-(p-tolyl)urea B-4-1

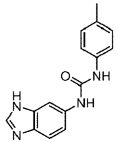

### Procedure 1: p-Nitrophenyl p-toluidine carbonate II-9-1

p-toluidine (21mg, 0.2 mmol) was mixed with 4-nitrophenylcarbonyl chloride (48 mg, 0.24 mmol), and triethanolamine (0.2 mL), dichloromethane (1 mL), and tetrahydrofuran (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (dichloromethane: hexane = 5: 1) to obtain p-nitrophenyl p-toluidine carbonate II-9-1 (42 mg, 77%), liquid phase mass spectrum m/z = 273.1[m+H]+.

### Procedure 2: Urea 1-(1H-Benzo[d]imidazol-6-yl)-3-(p-tolyl)-

p-Nitrophenyl p-toluenecarbamate (27 mg, 0.1 mmol) was mixed with 1H-benzo[d]imidazol-6-amine (16 mg, 0.12 mmol) and K₂CO₃ (0.2 g, 1.45 mmol), and acetonitrile (3 mL) were added, which was heated to 40°C and stirred for 4 h. After completion of the reaction (monitored by TLC), the reaction mixture was separated and concentrated in vacuo. The crude reaction mixture thus obtained was washed with dichloromethane, then EtOAc and finally MeOH (1 mL each). Finally, the reaction product was recrystallized using EtOAc (under warm conditions) to obtain urea 1-(1H-benzo[d]imidazol-6-yl)-3-(p-tolyl)- B-4-1 (20 mg, 75%), liquid mass spectrum m/z = 266.1 [m+H]+.

### Embodiment 32: 1-(1H-benzo[d]imidazole-6-yl)-3-(3-bromophenyl)urea B-4-2

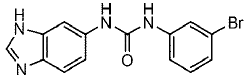

### Procedure 1: 4-Nitrophenyl(3-bromophenyl) aminocarbonate II-9-2

3-Bromoaniline (34 mg, 0.2 mmol) was mixed with 4-nitrophenylcarbonyl chloride (48 mg, 0.24 mmol), and triethanolamine (0.2 mL), dichloromethane (1 mL), and tetrahydrofuran (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (dichloromethane: hexane = 5:1) to obtain 4-nitrophenyl (3-bromophenyl) aminocarbonate II-9-2 (46 mg, 68%), liquid phase mass spectrum m/z = 338.1[m+H]+.

### Procedure 2: 1-(1H-benzo[d]imidazole-6-yl)-3-(3-bromophenyl)urea

4-nitrophenyl(3-bromophenyl)aminocarbonate (34 mg, 0.1 mmol) was mixed with 1H-benzo[d]imidazol-6-amine (16 mg, 0.12 mmol) and K₂CO₃ (0.2 g, 1.45 mmol), and acetonitrile (3 mL) was added, which was heated to 40° C and stirred for 4 h. After completion of the reaction (monitored by TLC), the reaction mixture was separated and concentrated in vacuo. The crude reaction mixture thus obtained was washed with dichloromethane, then EtOAc and finally MeOH (1 mL each). Finally, the reaction product was recrystallized using EtOAc (under warm conditions) to obtain urea, 1-(1H-benzo[d]imidazol-6-yl)-3-(3-bromophenyl)- B-4-2 (22 mg, 68%), liquid mass spectrum m/z = 330.0[m+H]+.

### Embodiment 33: N-Methyl-N-phenyl-1H-benzo[d]imidazole-6-carboxamide B-5

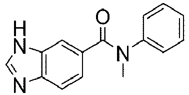

Its synthetic route is:

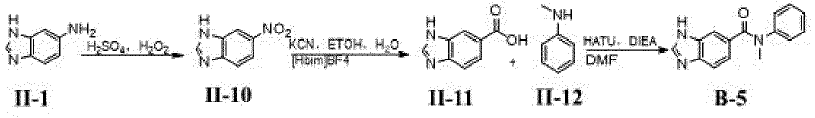

### Procedure 1: 6-Nitro-1H-benzo[d]imidazole 11-10

A solution of 1H-benzo[d]imidazol-6-amine (40 mg, 0.3 mmol) in concentrated sulfuric acid (100 µL) was added dropwise to a solution of H₂O₂ (240 mg, 2.2 mmol) in oleum (500 µL), and the reaction temperature was kept at 0°C. After stirring at 10-25 °C for 3 h, the reaction mixture was brought to pH = 11-12 by adding 40% aqueous NaOH at 0-5 °C. The obtained mixture was extracted with ethyl acetate. The combined organic layer was washed with saturated aqueous sodium chloride, dried over Na₂SO₄ and filtered. The solvent was distilled off under reduced pressure to obtain the desired 6-nitro-1H-benzo[d]imidazole II-10 (35 mg, 72%) LC-mS m/z = 163.0[m+H]+.

### Procedure 2: lH-Benzo[d]imidazole-6-carboxylic acid 11-11

6-Nitro-1H-benzo[d]imidazole (39 mg, 0.24 mmol) was mixed with KCN (195 mg, 3 mmol), 1-butylimidazolium tetrafluoroborate (3 mg, 0.014 mmol), EtOH (2 mL), and water (2 mL) were added, which was heated to 80°C and stirred for 19 h. Then 10 mL of water was added and the mixture was extracted with CH₂Cl₂ (3×5 mL) and ether (3x10 mL). Acidify to pH 1-2 with hydrochloric acid and extract with ether (3×10 mL). Magnesium sulfate (3 g) and activated carbon (1 g) were added and stirred for 5 h. The solid was filtered off, the filtrate was evaporated, and the residue was crystallized from the corresponding solvent to obtain the desired 1H-benzo[d]imidazole-6-carboxylic acid II-11 (14 mg, 40%) LC-mS m/z = 162.0 [m+H]+.

### Procedure 3: N-Methyl-N-phenyl-1H-benzo[d]imidazole-6-carboxamide

1H-benzo[d]imidazole-6-carboxylic acid (16 mg, 0.1 mmol) was mixed with N-methylaniline (13 mg, 0.12 mmol) and 2-(7-azabenzotriazol-1-yl)-N,N,N',N'-tetramethyluronium hexafluorophosphate (38 mg, 0.1 mmol), and N,N-diisopropylethylamine (0.2 mL) and N,N-dimethylformamide (2 mL) were added, which was stirred at room temperature for 18 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) and distilled off the solvent under reduced pressure to obtain N-methyl-N-phenyl-1H-benzo[d]imidazole-6-carboxamide B-5 (16 mg, 65%), liquid mass spectrum m/z = 251.1 [m+H]+.

### Embodiment 34: N-(Tetrahydro-2H-pyran-4-yl)-1H-benzo[d]imidazol-6-amine B-6-1

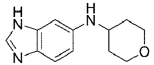

Procedure: 1H-Benzo[d]imidazol-6-amine (13 mg, 0.1 mmol) was mixed with tetrahydro-4H-pyran-4-one (12 mg, 0.12 mmol), methanol (2 mL) was added, which was stirred at room temperature for 2 h. Then sodium cyanoborohydride (20 mg, 0.3 mmol) was added and the mixture was stirred at room temperature for 2 h. Aqueous NaOH (10 mL, 0.3 mol/L) was then added to the mixture. The obtained mixture was extracted with DCM (20×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain N-(tetrahydro-2H-pyran-4-yl)-1H-benzo[d]imidazol-6-amine B-6-1 (16 mg, 75%) LC/MS m/z = 217.1[m+H]+.

### Embodiment 35: N-(pyridin-4-methyl)-1H-benzo[d]imidazol-6-amine B-6-2

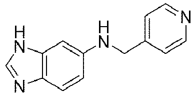

Procedure: 1H-benzo[d]imidazol-6-amine (13 mg, 0.1 mmol) was mixed with isonicotinaldehyde (13 mg, 0.12 mmol), and methanol (2 mL) was added, which was stirred at room temperature for 2 h. Then cyanoboron sodium hydride (20 mg, 0.3 mmol) was added and stirred at room temperature for 2 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain N-(pyridin-4-methyl)-1H-benzo[d]imidazol-6-amine B-6-2 (14 mg, 64%), liquid mass spectrum m/z = 224.1[m+H]+.

### Embodiment 36: n-cyclobutyl-1H-benzo[d]imidazol-6-amine B-6-3

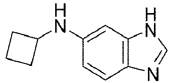

Procedure: 1H-benzo[d]imidazol-6-amine (13 mg, 0.1 mmol) was mixed with cyclobutanone (8 mg, 0.12 mmol), and methanol (2 mL) was added, which was stirred at room temperature for 2 h. Then sodium cyanoborohydride (20 mg, 0.3 mmol) was added and the mixture was stirred at room temperature for 2 h. Aqueous NaOH (10 mL, 0.3 mol/L) was then added to the mixture. The obtained mixture was extracted with DC m (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain n-cyclobutyl-1H-benzo[d]imidazol-6-amine B-6-3 (13 mg, 72%), liquid phase mass spectrum m/z = 187.1[m+H]+.

### Embodiment 37: 2-(1H-Benzo[d]imidazol-6-yl)isoindoline-1,3-dione B-7

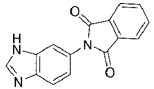

Its synthetic route:

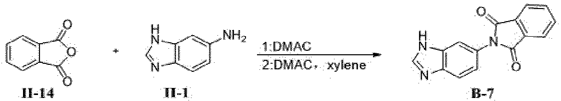

Procedure: 1H-benzo[d]imidazol-6-amine (13 mg, 0.1 mmol) was mixed with isobenzofuran-1,3-dione (18 mg, 0.12 mmol), and N,N-dimethylethyl amide (2 mL) was added, which was stirred at room temperature for 24 h. Then xylene (1 mL) was added and the mixture was stirred in an oil bath at 140°C for 48 h. After completion (monitored by TLC), the insoluble catalyst was isolated by filtration, washed with acetone and dried. The organic layer was concentrated under reduced pressure to obtain the desired product, which was washed with water, and recrystallized in ethanol. The crude product was purified by silica gel column chromatography (CH₂Cl₂/n-hexane = 1:1) and distilled off the solvent under reduced pressure to obtain 2-(1H-benzo[d]imidazol-6-yl)isoindoline-1,3-dione B-7(18 mg, 68%) LCMS m/z = 263.1[m+H]+.

### Embodiment 38: N-Phenyl-1H-benzo[d]imidazol-6-amine B-8

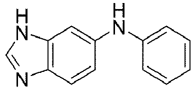

Its synthetic route:

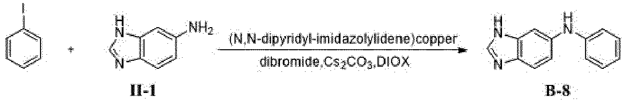

Procedure: 1H-benzo[d]imidazol-6-amine (13 mg, 0.1 mmol) was mixed with iodobenzene (24 mg, 0.12 mmol), (N,N-bipyridyl imidazolylidene) copper dibromide (10 mg, 0.023 mmol), and cesium carbonate (100 mg, 0.3 mmol), and 1,4-dioxane (5 mL) was added, which was stirred in an oil bath at 170°C for 12 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain N-phenyl-1H-benzo[d]imidazol-6-amine B-8 (17 mg, 82%), liquid phase mass spectrum m/z = 209.1 [m+H]+.

### Embodiment 39: (E)-6-Styryl-1H-benzo[d]imidazole B-9

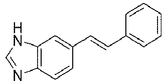

Its synthetic route:

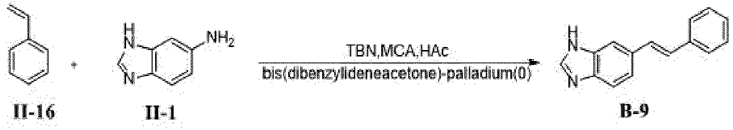

Procedure: 1H-Benzo[d]imidazol-6-amine (13 mg, 0.1 mmol) was mixed with styrene (13 mg, 0.12 mmol) and bis(dibenzylideneacetone)palladium(0) (5 mg, 0.0087 mmol), and nitroso tert-butyl ester (0.5 mL), chloroacetic acid (0.5 mL), and acetic acid (3 mL) were added, which was stirred in an oil bath at 50°C for 2 h. Aqueous NaOH (10 mL, 0.3 mol/L) was then added to the mixture. The obtained mixture was extracted with DCM (20 mL×3). The combined organic phases were dried over anhydrous Na₂SO₄, filtered and concentrated in vacuo. The crude product was purified by silica gel column chromatography (petroleum ether: ethyl acetate = 5:1) and distilled off the solvent under reduced pressure to obtain (E)-6-styryl-1H-benzo[d]imidazole B-9 (13 mg, 61%), liquid phase mass spectrum m/z = 220.1[m+H]+.

### Embodiment 40: N-(1H-Benzo[d]imidazol-6-yl)thiophene-2-sulfonamide B-10

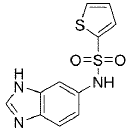

Its synthetic route:

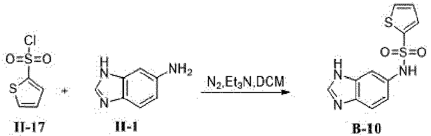

Procedure: 1H-Benzo[d]imidazol-6-amine (13 mg, 0.1 mmol) was mixed with thiophene-2-sulfonyl chloride (22 mg, 0.12 mmol), triethylamine (0.5 mL) and dichloromethane (3 mL) were added, which was stirred at room temperature for 24 h under nitrogen protection. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) and distilled off the solvent under reduced pressure to obtain N-(1H-benzo[d]imidazol-6-yl)thiophene-2-sulfonamide B-10 (13 mg, 45%), liquid phase mass spectrum m/z = 279.0[m+H]+.

### Embodiment 41 · N-(2-(1h benzo[d]imidazol-6-yl)aminoethyl)methylsulfonamide B-11

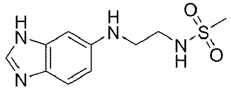

Its synthetic route:

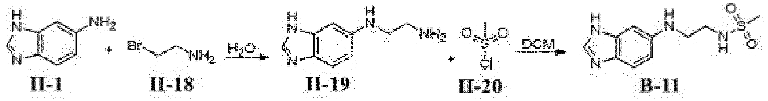

### Procedure 1: N1-(1H-Benzo[d]imidazol-6-yl)ethane-1,2-diamine II-19

1H-benzo[d]imidazol-6-amine (27 mg, 0.2 mmol) was mixed with 2-bromoethane-1-amine (29 mg, 0.24 mmol), and water (5 mL) was added, which was stirred in an oil bath at 95°C for 18 h. Ethyl acetate and water were added to the reaction mixture, and the organic layer was separated, which was dried over sodium sulfate and concentrated under reduced pressure. The mixture was purified by flash chromatography on silica gel (CH₂Cl₂methanol-ammonia) to obtain the desired product. The solvent was distilled off under reduced pressure to obtain N1-(1H-benzo[d]imidazol-6-yl)ethane-1,2-diamine II-19 (22 mg, 61%), liquid phase mass spectrum m/z = 176.1[m+H]+.

### Procedure 2: N-(2-(1h Benzo[d]imidazol-6-yl)aminoethyl)methylsulfonamide

N1-(1H-benzo[d]imidazol-6-yl)ethane-1,2-diamine (23 mg, 0.13 mmol) was mixed with methanesulfonyl chloride (28 mg, 0.16 mmol), and dichloromethane (5 mL) was added, which was stirred in an ice-water bath for 24 h. Ethyl acetate and water were added to the reaction mixture, and the organic layer was separated, which was dried over sodium sulfate and concentrated under reduced pressure. The residue was purified by silica gel column chromatography (ethyl acetate: hexane = 3:7) and distilled off the solvent under reduced pressure to obtain N-(2-(1Hbenzo[d]imidazol-6-yl)aminoethyl)methylsulfonamide B-11 (11 mg, 32%), liquid mass spectrum m/z = 254.1[m+H]+.

### Small molecule binding energy prediction:

The aminopyridine derivative molecules in Embodiments 1-21 and the benzimidazole derivative molecules in embodiments 22-41 were performed molecular docking with the OCT4 target protein by using AutoDock Vina and LeDock software, respectively, and 10 docking conformations were produced. The binding energy and ligand efficiency of each aminopyridine derivative molecule and each benzimidazole derivative molecule for optimal docking result with OCT4 target protein were calculated. Based on the docking results, the aminopyridine derivative molecule and benzimidazole derivative molecules were screened. The specific docking results are shown in Table 1 and Table 2, respectively: the second and third columns of the table are the binding free energy calculated by molecular docking software of AutoDock Vina and LeDock, respectively. The more negative the value, the stronger the binding ability of the small molecule ligand to the target protein. The fourth column of the table represents the ligand efficiency calculated by LeDock, and the larger the absolute value, the stronger the potential of small molecule activity. In the present invention, according to the independent algorithms of the two software, the binding energy level of the compounds involved in the present invention is predicted, and the predicted value shows that the binding energy of the compounds in the present invention is all far higher than the threshold value 3 set according to the target characteristics in the present invention.

**Table 1. Prediction of the binding of aminopyridine derivative molecules used in the present invention to target protein**

| Compound | AD_vina_binding_energy (kcal/mol) | Ledock_Energy (kcal/mol) | Ledock_Ligand_efficiency |
|---|---|---|---|
| A-1-1 | -5.9 | -4.04 | -0.22 |
| A-1-2 | -5.6 | -4.09 | -0.24 |
| A-2-1 | -5 | -3.98 | -0.25 |
| A-2-2 | -5.9 | -4.63 | -0.31 |
| A-2-3 | -6.4 | -4.86 | -0.24 |
| A-2-4 | -5.7 | -4.31 | -0.23 |
| A-2-5 | -5.95 | -4.33 | -0.2165 |
| A-2-6 | -5.3 | -4.03 | -0.22 |
| A-2-7 | -5.9 | -4.05 | -0.24 |
| A-3 | -6.8 | -3.98 | -0.22 |
| A-4-1 | -6.4 | -4.41 | -0.23 |
| A-4-2 | -5.4 | -4.83 | -0.25 |
| A-5 | -5.2 | -4.16 | -0.23 |
| A-6-1 | -5.4 | -3.95 | -0.26 |
| A-6-2 | -5.3 | -4.87 | -0.30 |
| A-6-3 | -5 | -3.97 | -0.31 |
| A-7 | -6.7 | -4.36 | -0.23 |
| A-8 | -5.6 | -4.11 | -0.27 |
| A-9 | -6.2 | -3.85 | -0.24 |
| A-10 | -5.2 | -4.79 | -0.28 |
| A-11 | -4.6 | -5.23 | -0.33 |

**Table 2. Prediction of the binding energy of the benzimidazole derivative molecule used in the present invention to targeting sequence**

| Compound | AD_vma_bindmg_energy (kcal/mol) | Ledock_Energy (kcal/mol) | Ledock_Ligand_efficiency |
|---|---|---|---|
| B-1-1 | -6.5 | -4.53 | -0.24 |
| B-1-2 | -5.7 | -4.13 | -0.23 |
| B-2 | -6.5 | -4.33 | -0.23 |
| B-3-1 | -5.3 | -4.68 | -0.28 |
| B-3-2 | -5.65 | -4.49 | -0.28 |
| B-3-3 | -6.8 | -4.52 | -0.22 |
| B-3-4 | -6 | -4.51 | -0.23 |
| B-3-5 | -6.2 | -4.72 | -0.22 |
| B-3-6 | -5.7 | -4.35 | -0.23 |
| B-4-1 | -6.45 | -4.46 | -0.22 |
| B-4-2 | -5.8 | -4.84 | -0.24 |
| B-5 | -6.3 | -4.23 | -0.22 |
| B-6-1 | -5.4 | -4.06 | -0.25 |
| B-6-2 | -5.8 | -4.95 | -0.29 |
| B-6-3 | -5.3 | -4.00 | -0.29 |
| B-7 | -6.5 | -4.51 | -0.23 |
| B-8 | -5.95 | -4.19 | -0.26 |
| B-9 | -6.6 | -4.31 | -0.25 |
| B-10 | -5.1 | -5.51 | -0.31 |
| B-11 | -4.8 | -5.36 | -0.32 |

### Differential verification of transcript expression caused by small molecules:

The purpose of the present invention is to use a highly selective activator to activate the target gene, and the important function of this type of activator is to activate the expression of OCT4, thereby increasing the expression abundance of OCT4 to downstream genes. Therefore, when verifying the function of the small molecule of the present invention, in addition to verifying the increased range of the OCT4 gene itself, the increase in the expression of the downstream gene Nanog of the OCT4 gene is also a functional verification index of the compound in the invention.

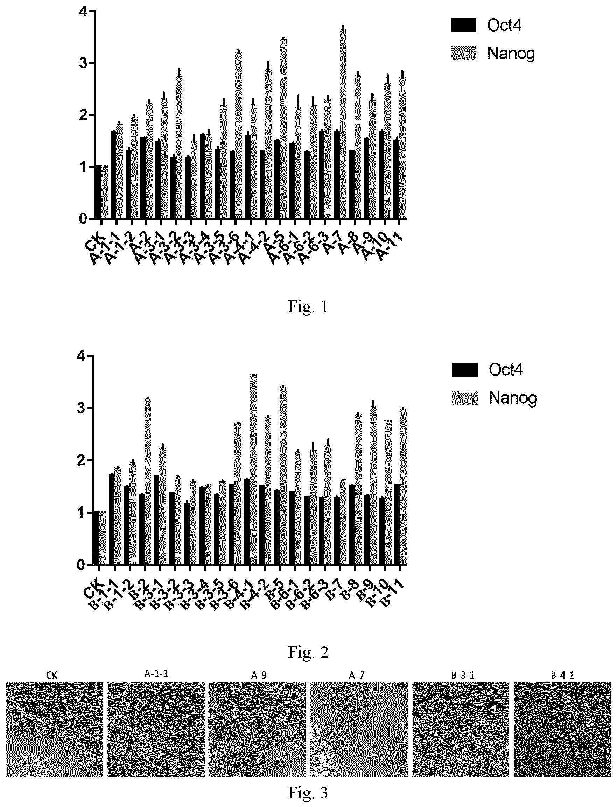

Culture human mesenchymal cells at T25, inoculate cells according to 4×10⁵, use serum-free Duchenne's modified Eagle's medium (DMEM-F12 medium), add 50 nM of the above-mentioned small molecules of aminopyridine derivatives and small molecules of benzimidazole derivatives, respectively Culture was carried out under the conditions of 37° C. and 5% carbon dioxide. On day 5, total RNA was extracted using RNeasy Mini or Micro Kit (QIAGEN), and cDNA was synthesized from 1 mg of RNA using SuperScript III First-Strand Synthesis System (Invitrogen). SYBR Premix Ex Taq (TaKaRa) and Thermal Cycler Dice Real Time System (TaKaRa) were used for Quan-titative PCR labeling and reaction, and beta-Actin was used as an internal reference. All data were analyzed by delta-Ct method. Each experiment was repeated three times, and variance statistics were performed. The primer sequences used to identify genes encoding different cell markers are shown in Table 3. The results are shown in Fig. 1 and Fig. 2. Compared with the control group without using small molecules, the above small molecules of aminopyridine derivatives and benzimidazole derivatives increased the basal expression of OCT4 and the expression of the downstream gene Nanog significantly.

**Table 3. Compound Effector Gene QPCR Primer Sequences**

| | |
|---|---|
| OCT4-F | CCATGCATTCAAACTGAGGT |
| OCT4-R | CCTTTGTGTTCCCAATTCCTT |
| Nanog-F | ACCTCAGCTACAAACAGGTGAA |
| Nanog-R | AAAGGCTGGGGTAGGTAGGT |
| βActin-F | GGCCGAGGACTTTGATTGCACA |
| βActin-R | GGGCACGAAGGCTCATCATTCAA |

### Early verification of cell pluripotency induced by small molecules:

The Rex1 gene is highly expressed in undifferentiated embryonic stem cells, and the in vitro culture of pluripotent stem cells is closest to the natural state of embryonic stem cells and preimplantation blastocysts in vivo (Boroviak, T et al., Nat. Cell Biol. 2014.16, 516- 528; Kalkan, T et al., Development. 2017, 144, 1221-1234). Expression of the Rex1 gene revealed that the cells were in a state of natural pluripotency similar to that of embryonic stem cells or live preimplantation blastocysts. Therefore, the present invention regards the expression of Rex1 as the terminal detection index of small molecule function.

The Human mesenchymal cells were cultured in T25, inoculated according to 4×10⁵, and cultured using serum-free Duchenne's modified Eagle's medium (DMEM-F12 medium), in which 50 nM of the above-mentioned aminopyridine derivative small molecule and benzimidazole derivative small molecule were added, respectively. The culture condition is 37°C and 5% carbon dioxide. Immunofluorescence staining identification was carried out on the fifth day: cells were fixed with 4% paraformaldehyde at room temperature for 40 min and washed twice with DPBS buffer; then it is permeabilized with 0.1% Triton X-100 for 5 min and washed twice with DPBS buffer; then the cells were incubated overnight at 4°C with DPBS buffer containing 10% horse serum and 0.1% Triton X-100; then anti-Rex1 primary antibody (Abeam, ab28141) diluted 1:200 in DPBS buffer with 2% horse serum and 0.1% Triton X-100 was added, and the cells were incubated at 37°C for 2 h, washed three times with DPBS buffer, then goat anti-rabbit IgG(H+L) diluted 1:100 in DPBS buffer with 2% horse serum and 0.1% Triton X-100 was added, photographed by Alexa Fluor 488 (Invitrogen A10037). As shown in Fig. 3, the chemical small molecule of the present invention can promote the mesenchymal cells to express the Rex1 gene and allow the mesenchymal cells to form pluripotent stem cell clone-like cell clusters.

## Claims

1. A compound of formula I structure: 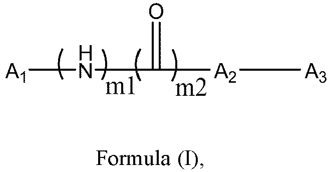 wherein:
A₁ is 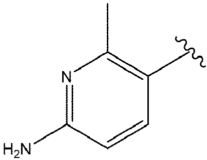 or 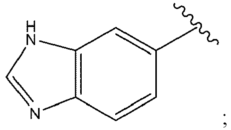
ml and m2 are 0 or 1, respectively;
A₂ is C1-C6 alkylene, C2-C6 alkenylene, -O(CH₂)q-, -NR₁-, -SO₂-, -(CH₂)_{V}NHS(O)₂-or a bond, wherein q is 1 or 2 or 3 or 4, V is 0 or 1 or 2, and R₁ is selected from H or C1-C4 alkyl;
A₃ is C1-C6 alkyl; C2-C6 alkenyl; C4-C6 cycloalkyl, wherein one carbon atom can be replaced by N, O, S heteroatom; 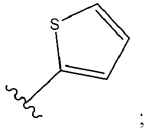 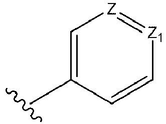 Z and Z₁ are N or CR₂, respectively, and R₂ is selected from H, halogen, C1-C4 alkyl or cyano; 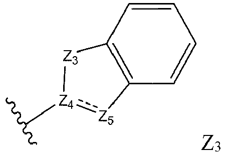 is N, O, S or C=O, when the bond between Z₄ and Z₅ is a single bond, Z₄ is N or CH, Z₅ is CH₂ or C=O, when the bond between Z₄ and Z₅ is a double bond, Z₄ is C, Z₅ is CH; and a pharmaceutically acceptable salt, solvate, active metabolite, polymorph, ester, optical isomer, prodrug thereof or a combination thereof.

2. The compound according to claim 1, which has the structure of formula (II) or formula (III): 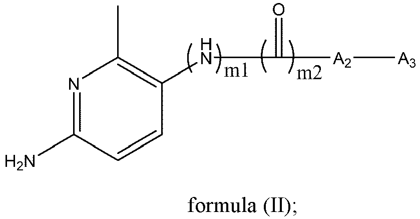 or

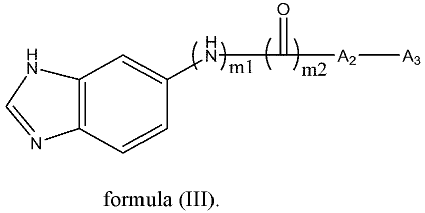

3. The compound according to claim 1 or 2, wherein:
A₂ is -CH₂-, -CH=CH-, -C(CH₃)=CH-, -O(CH₂)-, -O(CH₂)₂-, -NH-, -N(CH₃)-, -SO₂-, -NHS(O)₂-, -(CH₂)₂NHS(O)₂- or a bond.

4. The compound according to claim 3, wherein:
A₃ is -CH₃, butenyl, 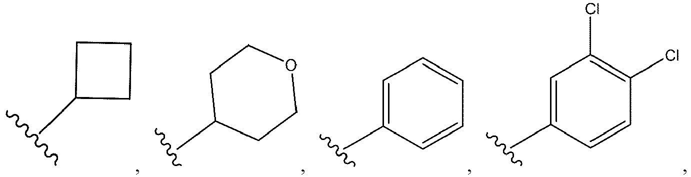 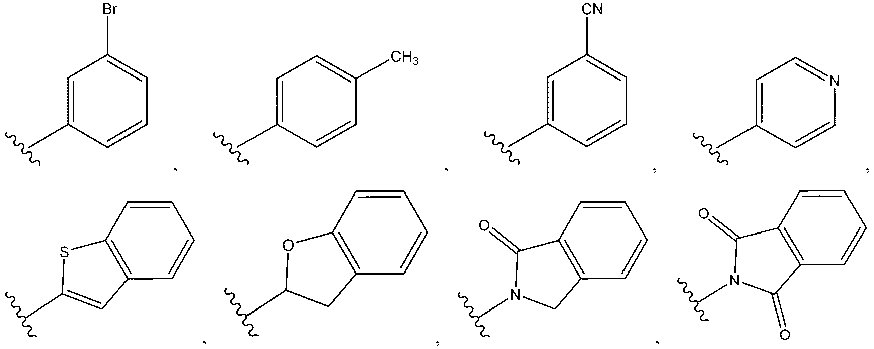 or

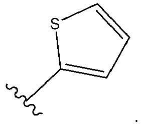

5. The compound according to claim 4, wherein:
m1 is 0, and m2 is 1;
A₂ is -N(CH₃)-;
A₃ is

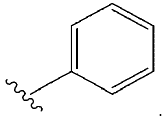

6. The compound according to claim 4, wherein:
m1 is 1, and m2 is 0;
A₂ is -CH₂-, -SO₂-, -(CH₂)₂NHS(O)₂- or a bond;
A₃ is -CH₃,

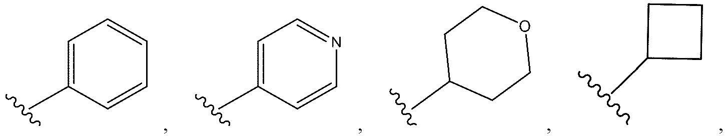

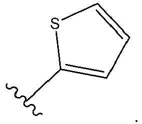

7. The compound according to claim 4, wherein:
m1 is 1, and m2 is 1;
A₂ is -CH₂-, -NH-, -C(CH₃)=CH- or a bond;
A₃ is -CH₃, -C(CH₃)=CH-CH₃, 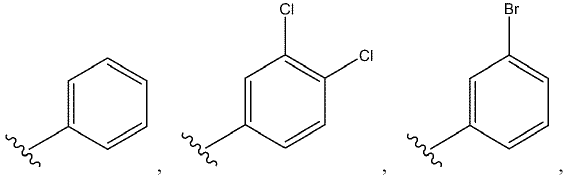 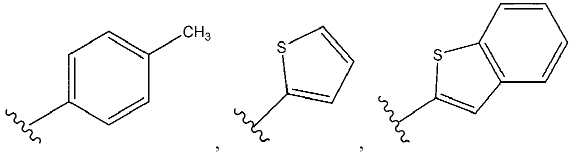 or

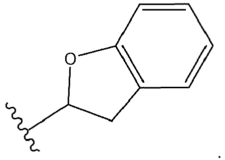

8. The compound according to claim 4, wherein:
m1 is 0, and m2 is 0;
A₂ is -CH₂-, -CH=CH-, -O(CH₂)-, -O(CH₂)₂- or a bond;
A₃ is 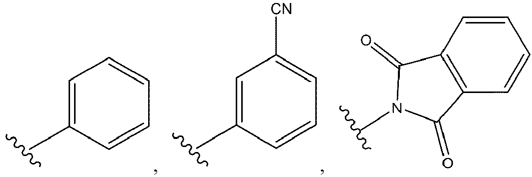 or

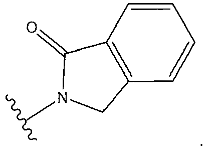

9. The compound according to claim 1, which is selected from:

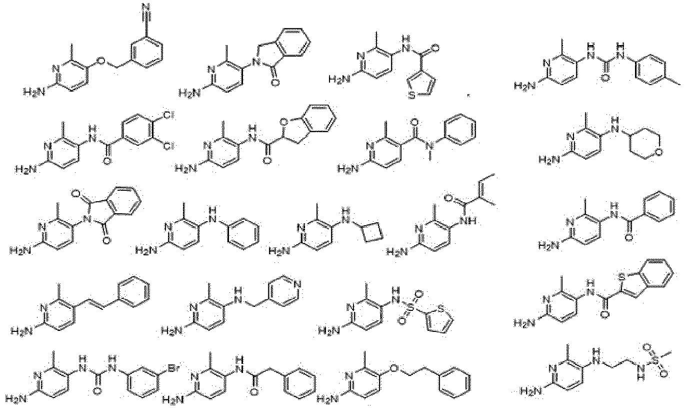

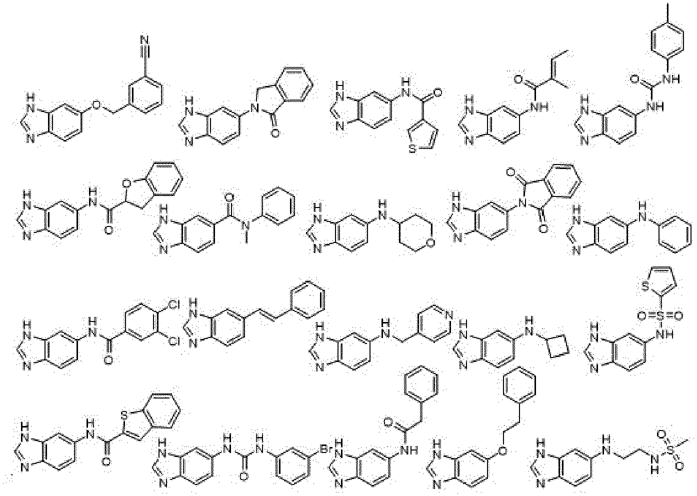

10. A pharmaceutical composition, comprising the compound of any one of claims 1 to 9, or a pharmaceutically acceptable salt, solvate, active metabolite, polymorph, ester, optical isomer or prodrug thereof, or any combination thereof; and at least one pharmaceutically acceptable carrier or excipient.

11. The said compound in any one of claims 1 to 9 and/or its pharmaceutically acceptable salt, solvate, active metabolite, polymorph, ester, optical isomer, prodrug or combination thereof in the use of preparing drugs of OCT4 high-selectivity activator of induced Pluripotent Stem Cells.

12. According to the use of claim 11, the diseases treated by OCT4 high-selectivity activator induced Pluripotent Stem Cells include cancer, heart disease, stroke, diabetes, obesity, Alzheimer's disease, Parkinson's disease, amyotrophic lateral cord sclerosis, myocardial infarction, muscular dystrophy, CMT-1A, spinal cord injury, traumatic brain injury, edentulousness, wound healing, bone marrow transplant, osteoarthritis, rheumatoid arthritis, alopecia, blindness, deafness, Crohn's diseases and genetic diseases and other similar diseases.

13. A method of obtaining induced Pluripotent Stem Cells in subjects with disease, comprising administering to the subject a therapeutically effective amount of the compound of any one of claims 1 to 9 and/or its pharmaceutically acceptable salt, solvate, active metabolite, polymorph, ester, optical isomer, prodrug, or combination thereof.

14. The method according to claim 13, wherein the subject with disease refers to Humans suffering from cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, edentulousness, wound healing, bone marrow transplantation, osteoarthritis, rheumatoid arthritis, alopecia, blindness, deafness, Crohn's disease and genetic diseases and other similar diseases.

15. A method of activating OCT4 function, comprising making the compound of any one of claims 1 to 9 and/or its pharmaceutically acceptable salt, solvate, active metabolite, polymorph, ester, optical isomer, prodrug, or combination thereof bind to the OCT4 target protein.
